# EUROPEAN PATENT APPLICATION

(11) **EP 4 574 213 A2**
(43) Date of publication of application: **25.06.2025**
(21) Application number: 25174118.7
(22) Date of filing: 09.05.2019
(51) Int. Cl.: A61P 31/20

(54) **LUBRICATED SUBSTRATES COMPRISING LAMBDA-CARRAGEENAN**

(30) Priority: 10.05.2018 US 201862669532 P
(62) Divisional of application: 24152265.5
(71) Applicant: Lanvira, LLC, New York 10022 (US)
(72) Inventor: TREMBLAY, Mario Elmen, St. Petersburg, 33701 (US)
(74) Representative: Keltie LLP

(57) **Abstract**

The present invention includes pourable, non-toxic lubricating compositions containing carrageenan and more precisely lambda carrageenan as an anti-viral agent, for protection from Human Papillomavirus (HPV), and methods of using the lubricating compositions as a non-oily, psuedoplastic lubrication product. Such methods include the lubrication of substrates, including condoms and sexual accessory devices, with the lambda-carrageenan-containing lubricants for contacting the skin of one or more sexual partners and reducing the propagation of HPV during sexual activity.

## Description

### FIELD OF THE INVENTION

The present invention relates to methods, kits, and lubricated substrates useful for preventing or reducing the propagation of human papillomavirus infections in humans.

### BACKGROUND OF THE INVENTION

According to the World Health Organization (WHO), human papillomavirus (HPV) is the most common sexually transmitted infection (STI) in the world, with over 14 million people acquiring new infections annually. In the United States alone, more than 42% of the people between the ages of 18 and 59 are infected with HPV, and 1 out of every 9 men are infected with oral HPV. Additionally, HPV is the root cause of essentially all cervical cancers, the second most common cancer in women worldwide by age-standardized incidence rate, leading to approximately 500,000 deaths per year. More than 85% of cervical cancer deaths are in developing countries, where it accounts for 13% of all female cancers. WHO also estimates that HPV causes 90% of anal cancer. In a separate study on HPV and throat cancer, AECOM found that the presence of an HPV type in the mouth increases the odds of developing head and neck cancer by twenty-two times. HPV is also the underlying cause of all genital warts.

HPV is spread from skin-to-skin contact, most commonly transmitted during sexual activity to the genitals, anus, and mouth. Consequently, condoms are only marginally effective in preventing transmission of HPV. While there are some treatment options for HPV, the reality is that there are more than 150 strains of HPV, approximately 30 of which have been shown to cause cancers and genital warts. Vaccines have been developed and can be useful-however, the best HPV vaccine available in the United States today only provides antiviral activity against 9 of the more than 150 HPV strains and they are only efficacious for a small subset of people. In 2015, the CDC reported that HPV vaccines only have an uptake rate 40% among adolescents, and that they are generally ineffective for people over 26 years old, as well as African-American women of any age. Further, the CDC has also reported that vaccines are also ineffective if the person has previously been exposed to HPV. Additionally, HPV vaccines are typically expensive, require multiple treatments or injections, and are largely unavailable to people in developing countries. As a result, a large proportion of people are unvaccinated and rely on condoms as their only protection against HPV.

Recently, personal lubricant compositions based on materials such as water gel networks, hydrogels, oil or silicone have been increasingly utilized in the art for enhancing intimate relationships and assisting couples during sexual activity. Some lubricants deliver more than the basic purpose of lubrication, including ones that deliver warming and cooling sensations, as well as some intended towards the application of massages. In particular, some lubricants include plant extracts, particularly sea-algal extracts, that are typically used for the benefit of hydration, or as a sensate (e.g. hot or cold feeling), or even to provide some form of epithelial healing. Typically, however, when an individual applies personal lubricant to the penis, the anus or to the vaginal mucosa, they do not typically have protection from venereal diseases that can propagate without protection, such as using condoms.

There have been some promising studies about the use of carrageenan formulations to reduce or inhibit the transmission of viruses *in vitro* (*see* Buck, C.B., et al., (2006) PLoS Pathogens 2 (7):671-680) and *in vivo* in mice (*see* Roberts, J.N., et al., (2007) Nature Medicine 13 (7):857-861, and Maguire, R.A., et al., (1998) Sexually Transmitted Diseases 25 (9):494-500). Several other patents and patent publications similarly discuss the use of carrageenan within medicaments as an antimicrobial or antiviral compound (*see* U.S. Pat. Nos. 5,208,031 and 8,367,098, and U.S. Pat. Pubs. 2005/0171053, 2005/0239742, 2005/0261240, 2006/0127340, 2008/0227749, 2009/0088405, and 2011/0229446, the disclosures of which are incorporated by reference in their entireties). Yet, there are very few commercially-available personal lubricants that contain carrageenan, because the addition of the carrageenan causes an exponential increase in the lubricant's viscosity and causes a loss in several of the rheological, tactile, and performance benefits gained by using a lubricant in conjunction with sexual activity. Particularly, carrageenan-based formulations typically dry quickly after being applied, resulting in sticky, non-lubricous residues that defeat the purpose of using a personal lubricant in the first place.

Consequently, there remains a need for a safe, effective lubricant formulation that can be applied before or during sexual activity, that allows both men and women to protect themselves in a pleasant and unobtrusive fashion from HPV infection. The lubricant formulation must balance efficacy against HPV while retaining the viscosity, tactility, and performance benefits by using personal lubricants during sexual activity. There also remains a need for a product that can be used in addition to a condom to provide the benefit of reducing the risk of transmitting HPV during sexual activity, particularly in encounters involving two or more men.

### SUMMARY OF THE INVENTION

The present invention provides personal lubricant compositions comprising lambda-carrageenan that are useful for reducing, inhibiting, ameliorating, or preventing the transmission, persistence, or symptoms caused by human papillomavirus (HPV). The reducing, inhibiting, ameliorating, or preventing the transmission of HPV includes such transmission through the epithelial or skin tissue of one or more people engaging in sexual contact. Accordingly, the antiviral lubricous compositions according to the present invention can be applied to any epithelial or skin tissue where HPV can reside or be spread, including the tissue within or on the cervix, vulva, vagina, penis, anus, mouth, or throat.

The present invention also provides methods for contacting a person's skin or epithelial tissue with the antiviral lubricous composition, particularly between people engaging in sexual activity. In some embodiments, contacting the skin or epithelial tissue of one or more sexual partners with the antiviral lubricous composition can be facilitated by the use of a substrate either before, during, or after sexual activity. Suitable substrates include objects or devices that can be inserted into body cavities such as the vagina, anus, and mouth during or in conjunction with sexual activity. Such objects or devices can include, but are not limited to, sexually-themed substrates such as condoms, vibrators, dildos, beads, and other sex toys, as well as non-sexually themed substrates that one or more users can nonetheless utilize during sexual activity, such as household items, food, and other objects.

In some embodiments, the antiviral lubricous compositions of the present invention are effective in reducing the transmission of HPV in humans, relative to placebo compositions that do not contain lambda-carrageenan. In further embodiments, the antiviral lubricous compositions provide protection against the transmission of HPV, including transmission that occurs during male-female sexual encounters, male-male sexual encounters, and female-female sexual encounters. In even further embodiments, the antiviral lubricous compositions provide protection against the transmission of HPV among high-risk sexual partners, including those who are already infected with at least one strain of HPV. In other further embodiments, the antiviral lubricous compositions provide protection against the transmission of HPV strains known to cause cancer, particularly cervical cancer.

In some embodiments, the antiviral lubricous compositions of the present invention comprise lambda-carrageenan that has been extracted from sea algae, particularly from the red algae, *Chondrus crispus.* In some embodiments, the carrageenan-containing sea algae extracts are water soluble, and lambda-carrageenan comprises at least 1% by weight of the sea algae extract, including at least about 25, 50, 60, 70, 80, or 85% by weight, up to at least about 90% by weight of the sea algae extract, particularly between about 80% by weight and about 90% by weight of the sea algae extract. In other embodiments, lambda-carrageenan comprises up to about 100% by weight of the sea algae extract, including up to about 99, 95, 90, 85, 80, 70, 60, 50, 25, 10, or up to about 2% by weight of the sea algae extract. In further embodiments, the lambda-carrageenan-containing sea algae extracts within the antiviral lubricous compositions comprise at least about 0.001% by weight of the antiviral lubricous composition, including at least about 0.01, 0.1, 1, or 3% by weight, up to at least about 5% by weight of the antiviral lubricous composition, particularly between about 1% by weight and about 3% by weight of the antiviral composition. In even further embodiments, the antiviral lubricous composition comprises between about 1.4% by weight to about 1.6% by weight of the sea algae extract. In still even further embodiments, the lambda-carrageenan-containing sea algae extracts within the antiviral lubricous compositions comprise up to about 10% of the antiviral lubricous composition, including up to about 5, 3, 2, 1, 0.1, or 0.05% by weight of the antiviral lubricous composition.

In some embodiments, the antiviral lubricous compositions of the present invention can be applied to one or more of the vagina, anus, mouth, or penis of one or more sexual partners either prior to sexual activity or afterward. In some embodiments, the antiviral lubricous composition is a pourable composition having a viscosity of less than about 10,000 cP, including less than about 8,000, 6,000, 4,000, or 2,000 cP, down to less than about 1,000 cP. In other embodiments, the antiviral lubricous composition has a viscosity of at least about 500 cP, including at least about 1,000, 2,000, 4,000, or 6,000 cP, up to at least about 8,000 cP. In further embodiments, the antiviral lubricous composition has a viscosity between about 500 cP and about 8,000 cP, more particularly between about 1,000 cP and about 4,000 cP, even more particularly between about 1,500 cP and about 2,500 cP, and still more particularly about 2,000 cP. In further embodiments, the antiviral lubricous composition is a non-Newtonian, pseudoplastic fluid that undergoes shear thinning in response to mechanical strain, for instance, when two or more people are engaging in sexual activity. In even further embodiments, the antiviral lubricous composition possesses a rheological profile such that the lubricity of a dried, antiviral lubricous composition on the skin can be retained upon adding water to the dried, antiviral lubricous composition.

In some embodiments, the antiviral lubricous compositions of the present invention can be prepared substantially free of components typically utilized in commercially-available personal lubricants, including but not limited to: oils, particularly silicone oils; cellulose; and polyquaterniums. In other embodiments, the antiviral lubricous compositions can be prepared to be substantially free of spermicides, such as nonoxynol-9, that are also often present commercially-available personal lubricants.

In some embodiments, antiviral personal lubricants of the present invention can simply comprise lambda-carrageenan-containing sea algae extract and water. In other embodiments, antiviral lubricous compositions can further comprise additional components in addition to lambda-carrageenan and water, either to supplement the composition's antiviral activity or to enhance its performance during sexual activity. Such compounds that can be added to personal lubricants to supplement or compliment their antiviral activity against HPV include, but are not limited to: polyols, salts, sweetening agents, aromatic agents, pH-adjusting agents, and preservatives.

In some embodiments, the antiviral lubricous compositions of the present invention can further comprise at least one polyol selected from the group consisting of glycerol, propylene glycol, polyethylene glycol, and polypropylene glycol, including combinations thereof, to be utilized as agents that enhance the sensation, tactility, and/or lubricity experienced by the wearer or his or her partners during sexual activity. In further embodiments, the polyol is propylene glycol, and comprises less than about 50% by weight of the antiviral lubricous composition, including less than about 25, 15, 10, 9, 8, 7, 6, 5, 4, 3, or 2% by weight, down to less than about 1% by weight of the antiviral lubricous composition. In other further embodiments, the antiviral lubricous composition comprises at least about 1% by weight of propylene glycol, including at least about 2, 3, 4, 5, 6, 7, 8, 9, 10, 15, or 25% by weight, including at least about 35% by weight. In even further embodiments, the antiviral lubricous composition comprises between about 2.0% and about 10.0% by weight of the antiviral lubricous composition. In still further embodiments, the antiviral lubricous composition comprises between about 8.0% and about 8.5% by weight of propylene glycol. In other still further embodiments, the antiviral lubricous composition comprises between about 4.0% and about 4.5% by weight of propylene glycol. In yet still further embodiments, the antiviral lubricous composition comprises between about 2.0% and about 2.5% by weight of propylene glycol.

In some embodiments, the antiviral lubricous compositions of the present invention can further comprise one or more sweetening agents, particularly saccharin, comprising about 0.01% by weight to about 5% by weight of the antiviral lubricous composition, particularly about 0.1% by weight to about 2% by weight of the antiviral lubricous composition.

In some embodiments, the antiviral lubricous compositions of the present invention can optionally further comprise one or more aromatic agents designed to provide a pleasing fragrant effect on the composition. Aromatic agents can include essential oils or component compounds within essential oils capable of imparting an odor. Non-limiting examples of fragrances that can be provided by such aromatic agents include citrus, lemon, berry, or peppermint fragrances. In some embodiments, aromatic agents can comprise between about 0.01% and about 5% by weight of the antiviral lubricous composition, particularly between about 0.1% and about 2% by weight of the antiviral lubricous composition.

In some embodiments, the antiviral lubricous compositions of the present invention can further comprise one or more pH-adjusting agents comprising an acid, particularly an organic acid, and more particularly citric acid. In further embodiments, the pH of the antiviral lubricous composition is less than about 8.0, including less than about 7.0, 6.5, 6.0, 5.5, 5.0, 4.5, or 4.0, down to less than about 3.5. In other further embodiments, the pH of the antiviral lubricous composition is at least about 3.5, including at least about 4.0, 4.5, 5.0, 5.5, 6.0, 6.5, or 7.0, up to at least about 8.0. In even further embodiments, the pH of the antiviral lubricous composition is between about 5.5 and about 7.0. In still further embodiments, the pH of the antiviral lubricous composition can be optimized to be either applied to the vagina directly, or to contact the vagina during sexual activity. In such embodiments, the pH of the antiviral lubricous composition is between about 3.5 and about 5.5, particularly about 4.5.

In some embodiments, the antiviral lubricous compositions of the present invention can further comprise between about 0.01% by weight and about 1.0% by weight of one or more preservatives, particularly one or more preservatives selected from the group consisting of 2-phenoxylethanol, chlorphenesin, and sodium dehydroacetate, including combinations thereof.

In some embodiments, the antiviral lubricous compositions of the present invention can further comprise a salt, particularly a sodium salt or a zinc salt, more particularly a zinc salt, that can be utilized to increase the ionic strength of the composition while also supporting or complementing either or both of the rheological properties or antiviral activity of the composition.

In some embodiments, the antiviral lubricous compositions are made by a process comprising the steps of: (a) providing a water-soluble sea algae extract comprising lambda-carrageenan, wherein the sea algae extract comprises at least 50% by weight lambda-carrageenan; and (b) mixing the water-soluble sea algae extract, at least one polyol, and a pH-adjusting agent comprising an acid, with an aqueous solution, to form a lubricous composition comprising at least about 0.001% by weight the sea algae extract, wherein the antiviral lubricous composition has a viscosity of less than about 10,000 cP.

In some embodiments, methods for reducing, inhibiting, ameliorating, or preventing the transmission of HPV between partners engaging in sexual activity comprise the steps of: (A) providing any of the above-described antiviral lubricous compositions; (B) providing a substrate comprising at least one skin-contacting surface and configured for insertion into a body cavity, particularly the vagina, mouth, or anus, of one or more of the partners; (C) lubricating the at least one skin-contacting surface of the substrate with the antiviral lubricous composition, thereby producing a lubricated substrate; (D) contacting the lubricated substrate with the skin or epithelial tissue of one or more of the partners, particularly skin or epithelial tissue located on or within at least one of the vagina, anus, mouth, or penis; and (E) transferring the antiviral lubricous composition from the lubricated substrate to at least one of the vagina, anus, mouth, or penis of one or more of the partners, thereby reducing, inhibiting, ameliorating, or preventing the transmission of HPV. In further embodiments, at least one partner is male and at least one partner is female. In other further embodiments, at least two partners are male. In still other further embodiments, at least two partners are female. In alternate embodiments, there is only a single person, either a man or a woman, using the substrate during sexual activity. In other alternative embodiments, there are two or more sexual partners, where each of the sexual partners can be either a man or a woman.

In some embodiments, methods for reducing, inhibiting, ameliorating, or preventing the transmission of HPV between partners engaging in sexual activity comprise the steps of: (A) providing a pourable, homogeneous antiviral lubricous composition made by a process comprising the steps of: (i) providing a water-soluble sea algae extract comprising lambda-carrageenan, wherein the sea algae extract comprises at least 50% by weight lambda-carrageenan; and (ii) mixing the water-soluble sea algae extract and a pH-adjusting agent comprising an acid, with an aqueous solution, to form a lubricous composition comprising at least about 0.001% by weight the sea algae extract, wherein the antiviral lubricous composition has a viscosity of less than about 10,000 cP; (B) providing a substrate comprising at least one skin-contacting surface and configured for insertion into a body cavity, particularly the vagina, mouth, or anus, of one or more of the partners; (C) lubricating the at least one skin-contacting surface of the substrate with the antiviral lubricous composition, thereby producing a lubricated substrate; (D) contacting the lubricated substrate with the skin or epithelial tissue of one or more of the partners, particularly skin or epithelial tissue located on or within at least one of the vagina, anus, mouth, or penis; and (E) transferring the antiviral lubricous composition from the lubricated substrate to at least one of the vagina, anus, mouth, or penis of one or more of the partners, thereby reducing, inhibiting, ameliorating, or preventing the transmission of HPV.

In some embodiments, the skin-contacting surface is any surface of the substrate that is configured to contact the skin or epithelial tissue of one or more of the partners. In further embodiments, the skin-contacting surface of the substrate is configured to contact the skin or epithelial tissue of the user of the substrate him- or herself. In other further embodiments, the skin-contacting surface of the substrate is configured to contact the skin or epithelial tissue of the user's sexual partner(s). In even further embodiments, the skin-contacting surface of the substrate is configured to contact the skin or epithelial tissue of the user and the user's sexual partner(s).

In some embodiments, the substrate is a condom. In further embodiments, the method further comprises the steps of placing a condom over a penis of one partner, lubricating the condom with the antiviral lubricous composition *in situ*, and contacting the skin or epithelial tissue of one or more additional partners. In other further embodiments, the method further comprises the steps of sealing the lubricated condom within a packaging, storing the lubricated condom within the packaging, and removing the lubricated condom from the packaging prior to contacting the skin or epithelial tissue of one or more of the partners.

In other embodiments, the substrate is a sexual accessory device including but not limited to sex toys, dildos, vibrators, rings, or beads. In further embodiments, the method further comprises the steps of sealing the lubricated sexual accessory device within a packaging, storing the lubricated sexual accessory device within the packaging, and removing the lubricated sexual accessory device from the packaging prior to contacting the skin or epithelial tissue of one or more of the partners. The antiviral lubricous composition can be applied to any skin-contacting surface of the sexual accessory device, typically prior to contact with the skin of one or more the sexual partners, particularly prior to insertion into the vagina, anus, or mouth.

In some embodiments, a condom lubricated by any of the antiviral lubricous compositions of the present invention can be applied to an unlubricated sexual accessory device to provide lubricity for use during sexual activity. In other embodiments, an unlubricated condom can be applied to a sexual accessory device lubricated by any of the antiviral lubricous compositions of the present invention. In still other embodiments, an unlubricated condom can be applied to an unlubricated sexual accessory device and subsequently be lubricated by any of the antiviral lubricous compositions of the present invention.

The present invention also provides kits for reducing, inhibiting, or ameliorating the transmission of HPV between partners engaging in sexual activity, the kit comprising any of the above-described antiviral lubricous compositions and instructions describing any of the methods disclosed above for contacting the antiviral lubricous composition with the skin of one or more of the partners. Kits can also include one or more substrates as described above. In some embodiments, the substrate is a condom. In further embodiments, the condom is pre-lubricated. In even further embodiments, condoms pre-lubricated with the antiviral lubricous compositions of the present invention can be stored for weeks, months, or years, consistent with the storage times of pre-lubricated condoms in which the lubricant does not contain carrageenan.

These and other embodiments of the present invention will be apparent to one of ordinary skill in the art from the following detailed description.

### BRIEF DESCRIPTION OF THE FIGURES

Figure 1 shows a plot of the viscosity of antiviral lubricous compositions as a function of concentration of carrageenan.
Figure 2 shows the trial profile for an *in vivo* study assessing the efficacy of an antiviral lubricant composition in inhibiting transmission of cervical HPV.
Figure 3 shows the distribution of actual time between enrollment and subsequent visits relative to the timing of schedule visits during the *in vivo* study trial period.
Figure 4A shows the cumulative incidence of a first new HPV infection detected during the *in vivo* study trial period.
Figure 4B shows the cumulative incidence of all new HPV infections of any time detected during the *in vivo* study trial period.
Figure 5 shows sub-group analyses of the first occurrence of newly-detected HPV infections acquired by study participants.

### DETAILED DESCRIPTION OF THE INVENTION

It should be understood that while reference is made to exemplary embodiments and specific language is used to describe them, no limitation of the scope of the invention is intended. Further modifications of the methods described herein, as well as additional applications of the principles of those inventions as described, which would occur to one skilled in the relevant art and having possession of this disclosure, are to be considered within the scope of this invention. Furthermore, unless defined otherwise, all technical and scientific terms used herein have the same meaning as commonly understood by one of ordinary skill in the art to which embodiments of this particular invention pertain. The terminology used is for the purpose of describing those embodiments only, and the terminology is not intended to be limiting unless specified as such. Headings are provided for convenience only and are not to be construed to limit the invention in any way. Additionally, throughout the specification and claims, a given chemical formula or name shall encompass all optical isomers and stereoisomers, as well as racemic mixtures where such isomers and mixtures exist.

The present disclosure includes aqueous compositions that are active against human papillomavirus (HPV) *in vivo,* and which can be utilized as personal lubricants during sexual activity. The antiviral lubricous compositions can be contacted with the skin or epithelial tissue anywhere an HPV infection is known to be present, or in areas that are commonly lubricated during sexual activity, including but not limited to the vagina, anus, penis, and mouth. The antiviral lubricous compositions are capable of reducing, inhibiting, ameliorating, or preventing the transmission and/or the persistence of HPV between sexual partners, including male-female, male-male, and female-female sexual partnerships.

The anti-HPV activity of the antiviral lubricous compositions of the present invention results from the presence of carrageenan, particularly lambda carrageenan. Carrageenan is a generic term for a broad family of naturally occurring sulfated polysaccharides that are extracted from a wide range of species of seaweed algae, particularly from *Chondrus crispus*, a red seaweed found on the Atlantic coast of the United States. Extracted carrageenans can typically be obtained in one of ten forms, which differ in terms of sulfation content and acid/base character. Carrageenans are commonly used in the food and pharmaceutical industry as thickening agents, and most carrageenan forms are inactive against HPV. However, the lambda-carrageenan form has been shown to be active against HPV *in vitro* (see Buck, C.B., *et al,* above) and *in vivo* (see the "Examples" section, below). Several carrageenan extracts contain the lambda-form of carrageenan, particularly Viscarin^{®} PC 209, a powdered extract available from the FMC Corporation. In some embodiments, the sea algae extract comprising lambda-carrageenan and utilized to synthesize the antiviral lubricous compositions of the present invention is Viscarin^{®} PC 209. Mixing the powdered sea algae extract with water and heating it results in a non-Newtonian, pseudoplastic fluid that is homogenous and can be modified to possess rheological characteristics that create a satisfying experience when used a personal lubricant.

Without being bound by any particular theory, the lambda-carrageenan employed in the antiviral lubricous compositions of the present invention utilize a "lock and key" type mechanism by which the polysaccharide attracts the HPV virus, interacts with viral capsid, and then completely arrests and prevents the replication of the HPV virus in humans. This results in significant reduction of the transmission and persistence of the HPV virus in sexually active men and women. Human clinical studies using compositions comprising lambda-carrageenan described below demonstrate efficacy in inhibiting and/or preventing HPV infection in humans, including significantly blocking transmission of various HPV infection, including genital warts and cancer-causing HPV infections.

Although carrageenans have historically been used as thickening agents, they generally are not preferred in personal lubricant formulations because they cause an exponential increase in the viscosity of the composition, which results in an unsatisfying sensation and performance during sexual activity. As a result, topical carrageenan-containing compositions that are used to treat HPV are typically gels or creams that have thicker viscosities (see U.S. Pat. Pub. 2005/0261240, above). Such compositions tend to dry out quickly once applied to the skin or epithelial tissue, causing it to form a sticky residue and lose its lubricity, a situation that is particularly undesirable during sexual activity. On the other hand, compositions that are successful as personal lubricants and retain their lubricity commonly comprise compounds that inherently increase the lubricity of the composition, such as oils, particularly silicone oils, gums, celluloses, glycerins, polyols, glycols, glycans, polyquaterniums, and other polymers. While these lubricants do provide pleasing results during sexual activity, they do not provide any protection against HPV.

The antiviral lubricous compositions of the present invention are unique in that they have the ability to inhibit HPV while at the same time possessing properties that support their performance as lubricants that are used during sexual activity. Combating the unfavorable rheological properties typically exhibited in carrageenan-containing compositions can be mediated in part by utilizing sea algae extracts, for example, Viscarin^{®} PC 209, that contain a relatively high ratio of lambda-carrageenan relative to the other forms of carrageenan present in the extract. Without being bound by a particular theory, as opposed to the other carrageenans, which form a gel network upon mixing with water, lambda-carrageenan instead forms a non-Newtonian fluid. The result is a pourable, psuedoplastic composition that undergoes shear thinning in response to mechanical strain, for instance, when one, two, or more people are engaging in sexual activity. Thus, in some embodiments, lambda-carrageenan comprises at least 1% by weight of the sea algae extract, including at least about 25, 50, 60, 70, 80, or 85% by weight, up to at least about 90% by weight of the sea algae extract, particularly between about 80% by weight and about 90% by weight of the sea algae extract. In other embodiments, lambda-carrageenan comprises up to about 100% by weight of the sea algae extract, including up to about 99, 95, 90, 85, 80, 70, 60, 50, 25, 10, or up to about 2% by weight of the sea algae extract. In further embodiments, the lambda-carrageenan-containing sea algae extracts within the antiviral lubricous compositions comprise at least about 0.001% by weight of the antiviral lubricous composition, including at least about 0.01, 0.1, 1, or 3% by weight, up to at least about 5% by weight of the antiviral lubricous composition, particularly between about 1% by weight and about 3% by weight of the antiviral composition. In even further embodiments, the antiviral lubricous composition comprises between about 1.4% by weight to about 1.6% by weight of the sea algae extract. In still further embodiments, the sea algae extract is Viscarin^{®} PC 209.

Similarly, the rheology, sensation, and overall performance of the lambda-carrageenan-containing, antiviral lubricous composition can be controlled by the addition of a minor concentration of a polymer, particularly a polyol, to the antiviral lubricous composition. As described above, polyols are one class of compounds that are commonly found in commercially-available personal lubricant products. Although polyols are typically added to enhance the solubility of other polymers that might be present in commercial products, polyols can also be added in small quantities to antiviral lubricous compositions of the present invention to decrease the viscosity, provide a secondary source of lubricity, and/or to provide a stimulating "warming" or "tingling" feeling that is often pleasing to the user during sexual activity. Non-limiting examples of polyols that can be added can include glycerol, propylene glycol, polyethylene glycol, and polypropylene glycol, including combinations thereof. In some embodiments, the polyol is a short-chain, non-polymeric compound such as propylene glycol. In further embodiments, the propylene glycol can comprise less than about 50% by weight of the antiviral lubricous composition, including less than about 25, 15, 10, 9, 8, 7, 6, 5, 4, 3, or 2% by weight, down to less than about 1% by weight of the antiviral lubricous composition. In other further embodiments, the antiviral lubricous composition comprises at least about 1% by weight of propylene glycol, including at least about 2, 3, 4, 5, 6, 7, 8, 9, 10, 15, or 25% by weight, including at least about 35% by weight. In even further embodiments, the antiviral lubricous composition comprises between about 2.0% and about 10.0% by weight of the antiviral lubricous composition. In still further embodiments, the antiviral lubricous composition comprises between about 8.0% and about 8.5% by weight of propylene glycol. In other still further embodiments, the antiviral lubricous composition comprises between about 4.0% and about 4.5% by weight of propylene glycol. In yet still further embodiments, the antiviral lubricous composition comprises between about 2.0% and about 2.5% by weight of propylene glycol.

The addition of the polyol to the solubilized lambda-carrageenan-containing sea algae extract results an antiviral lubricous composition that is superior to known personal lubricant compositions because it maintains all of the sexual performance benefits of the commercially-available personal lubricants, while also having the ability to inhibit the transmission and/or persistence of HPV. Additionally, the antiviral lubricous compositions are thin enough to provide the tactile benefits of a personal lubricant composition, while also thick enough to remain on the skin or epithelial tissue, particularly the vagina, anus, penis, or mouth prior to initiating sexual contact. In some embodiments, the personal lubricant composition has a viscosity of less than about 10,000 cP, including less than about 8,000, 6,000, 4,000, or 2,000 cP, down to less than about 1,000 cP. In other embodiments, the antiviral lubricous composition has a viscosity of at least about 500 cP, including at least about 1,000, 2,000, 4,000, or 6,000 cP, up to at least about 8,000 cP. In further embodiments, the antiviral lubricous composition has a viscosity between about 500 cP and about 8,000 cP, more particularly between about 1,000 cP and about 4,000 cP, even more particularly between about 1,500 cP and about 2,500 cP, and still more particularly about 2,000 cP.

In addition, several supplemental components can be added to the antiviral lubricous composition to either complement the antiviral activity of the composition or to enhance the composition's performance during sex. In some embodiments, the pH of the antiviral lubricous composition can be controlled by the addition of a pH-adjusting agent. Typically, the pH of the composition once the lambda-carrageenan is solubilized into water is around 7 to 9. However, the effectiveness of the antiviral lubricous composition can be supplemented by controlling the pH of the composition to either match or be similar to the target surface to which the composition is applied. For instance, the pH of a healthy vagina typically ranges from about 3.5 to about 5.5, whereas the pH of other epithelial cells, including those located within the rectum, is closer to a neutral pH. Thus, in some embodiments, the pH-adjusting agent is an acid that is capable of reducing the pH to a range that is complementary to the intended epithelial surface, particularly below a pH of about 8.0, including less than about 7.0, 6.5, 6.0, 5.5, 5.0, 4.5, or 4.0, down to less than about 3.5. In other embodiments, the pH of the antiviral lubricous composition is at least about 3.5, including at least about 4.0, 4.5, 5.0, 5.5, 6.0, 6.5, or 7.0, up to at least about 8.0. In further embodiments, the pH of the antiviral lubricous composition is between about 3.5 and about 5.5, particularly about 4.5. In other further embodiments, the pH of the antiviral lubricous composition is between about 5.5 and about 7.0.

In some embodiments, the pH-adjusting agent is a strong acid, including, but not limited to, hydrochloric acid or sulfuric acid. However, in other embodiments, the pH-adjusting agent is a weak acid, in order to create a buffered antiviral lubricous composition. Weak acids can be selected based on their buffering capacity, pKa, and availability. Non-limiting examples of weak acids that can be utilized as pH-adjusting agents can include citric acid, lactic acid, and acetic acid. In further embodiments, the pH-adjusting agent is citric acid. In even further embodiments, the antiviral lubricous composition comprises between about 0.01% and about 1.0% by weight of citric acid, particularly between about 0.04% and about 0.06% by weight of citric acid.

In some embodiments, the antiviral lubricous composition can optionally further comprise one or more preservatives, which can be added to prevent microbial growth within the antiviral lubricous composition during storage. Any one or more of several preservatives may be selected from preservatives known to those of skilled in the art, including but not limiting to one or more of the following: methylparaben, benzoic acid, salicylic acid, sorbic acid, propylparaben, and sodium dehydroacetate, including combinations thereof. The preservative may be present in the compositions of this invention in an amount from about 0.01% to about 0.99% by weight of the composition.

In some embodiments, the antiviral lubricous composition can optionally further comprise one or more sweetening agents that enhance the flavor the composition when it comes in contact with a person's mouth. In some embodiments, the sweetening agent is an artificial sweetener, the presence of which can also inhibit bacterial growth within the antiviral lubricous composition during storage. Non-limiting examples of artificial sweeteners include, but are not limited to aspartame, saccharin, sucralose, neotame, and acesulfame potassium. In further embodiments, the sweetening agent is saccharin. In even further embodiments, the antiviral lubricous composition further comprises between about 0.1% to about 3% by weight of saccharin.

In addition to or in lieu of sweetening agents, the antiviral lubricous composition can optionally further comprise other aromatic agents or fragrances that can mask either the scent of the composition itself or the skin or epithelial tissue to which the antiviral lubricous composition is applied. Non-limiting examples of such fragrances include vanilla, lavender, oregano, thyme, lemongrass, lemons, oranges, anise, cloves, aniseed, cinnamon, geraniums, roses, mint, peppermint, citronella, eucalyptus, sandalwood, cedar, rosmarin, pine, vervain fleagrass, or ratanhiae, including combinations thereof, although any essential oil-based fragrance can be chosen. In other embodiments, the antiviral lubricous composition can optionally further comprise one or more chemical, aroma-causing compounds that cause the odor or fragrance within an essential oil-based fragrance. Non-limiting examples of chemical, aroma-causing compounds that can be comprised in any of the antiviral lubricous compositions include carvacrol, eugenol, linalool, thymol, *p*-cymene, myrcene, borneol, camphor, caryophillin, cinnamaldehyde, geraniol, nerol, citronellol, and menthol, including combinations thereof

In some embodiments, the antiviral lubricous composition can optionally further comprise one or more metal salts. The addition of a metal salt can have several effects, including controlling the ionic strength of the composition, enhancing its antimicrobial or antiviral activity, or adding in the solubilization of one or more components. Metal salts that can be added include, but are not limited to, zinc, silver, copper, alkali, or alkaline earth metal salts. In further embodiments, the metal salt is a zinc salt or a sodium salt.

In some embodiments, the antiviral lubricous composition can optionally further comprise one or more pharmaceutical antiviral, antifungal, or antimicrobial compounds.

In some embodiments, methods for reducing, inhibiting, or ameliorating the transmission of HPV between partners engaging in sexual activity comprise the steps of: (A) providing any of the above-described antiviral lubricous compositions; (B) providing a substrate comprising at least one skin-contacting surface and configured for insertion into a body cavity, particularly the vagina, mouth, or anus, of one or more of the partners; (C) lubricating the at least one skin-contacting surface of the substrate with the antiviral lubricous composition, thereby producing a lubricated substrate; (D) contacting the lubricated substrate with the skin or epithelial tissue of one or more of the partners, particularly skin or epithelial tissue located on or within at least one of the vagina, anus, mouth, or penis; and (E) transferring the antiviral lubricous composition from the lubricated substrate to at least one of the vagina, anus, mouth, or penis of one or more of the partners, thereby reducing, inhibiting, ameliorating, or preventing the transmission of HPV. In further embodiments, at least one partner is male and at least one partner is female. In other further embodiments, at least two partners are male. In still other further embodiments, at least two partners are female. In alternate embodiments, there is only a single person, either a man or a woman, using the substrate during sexual activity. In other alternative embodiments, there are two or more sexual partners, where each of the sexual partners can be either a man or a woman.

In some embodiments, the antiviral lubricous composition can be contacted with the skin, particularly skin located on or within at least one of the vagina, anus, mouth, or penis, of one or more of the sexual partners prior to sexual activity, in order to prophylactically inhibit the transmission of HPV from one sexual partner to another. In such embodiments, the antiviral lubricous composition can be contacted with the skin of one or more of the sexual partners less than about 8 hours, 4 hours, 2 hours, 1 hour, 30 minutes, 15 minutes, 5 minutes, 1 minute, or about 30 seconds prior to sexual activity, down to less than about 1 second prior to sexual activity. In other embodiments, the antiviral lubricous composition can be contacted with the skin of one or more of the sexual partners at least about 30 seconds, 1 minute, 5 minutes, 15 minutes, 30 minutes, 1 hour, 2 hours, 4 hours, or about 8 hours prior to sexual activity.

In some embodiments, the antiviral lubricous composition can be contacted with the skin, particularly skin located on or within at least one of the vagina, anus, mouth, or penis, of one or more of the sexual partners after sexual activity, in order to reduce the spread of HPV from cell to cell after HPV has been transmitted through skin-to-skin contact. In such embodiments, the antiviral lubricous composition can be contacted with the skin of one or more of the sexual partners less than about 8 hours, 4 hours, 2 hours, 1 hour, 30 minutes, 15 minutes, 5 minutes, 1 minute, or about 30 seconds after sexual activity, down to less than about 1 second after sexual activity. In other embodiments, the antiviral lubricous composition can be contacted with the skin of one or more of the sexual partners at least about 30 seconds, 1 minute, 5 minutes, 15 minutes, 30 minutes, 1 hour, 2 hours, 4 hours, or about 8 hours after sexual activity

Substrates suitable for use in conjunction with methods of the present invention include any object, device, or accessory that can be used to contact internal or external surfaces on or within the vagina, anus, mouth, or penis during sexual activity. Non-limiting examples of substrates that can be utilized in accordance with methods of the present invention include condoms or sexual accessory devices such as sex toys, dildos, vibrators, rings, and beads, comprising materials including but not limited to rubber, latex, plastic, wood, and/or metal. Such examples are described in U.S. Patent Nos. 6,983,751 and 9,119,763; U.S. Design Patent No. D599486, and U.S. Patent Publication 2006/0178602, the disclosures of which are included by reference in their entireties. Those skilled in the art would understand that there are countless other examples of substrates, both commercially available and improvised, sexually-themed or not, which can be utilized during sexual activity and to which antiviral lubricous compositions of the present invention can be applied.

In some embodiments, the substrate is a condom. The antiviral lubricous composition can be applied to at least one of an internal surface of the condom or an external surface of the condom, either prior to placing the condom over the penis, finger(s), or other body part or object, or afterward. In further embodiments, a condom pre-lubricated with any of the antiviral lubricous compounds of the present invention can be provided in a packaging that encloses the lubricated condom and seals it from an external environment outside of the packaging. In even further embodiments, the packaging comprises a watertight seal, thereby preventing loss of the antiviral lubricous composition before opening the packaging and applying the pre-lubricated condom over the penis or an analogous sexual accessory device and engaging in sexual activity. Methods for lubricating condoms and packaging them so they are protected from the outside environment prior to use during sexual activity are well known in the art.

Without being limited by a particular theory, the carrageenan-containing antiviral lubricous compositions of the present invention are uniquely suited for pre-packaged, pre-lubricated condoms relative to other carrageenan-containing compositions that are typically used to treat HPV because those compositions typically have a much higher viscosity and would not be able to withstand extended storage-weeks, months, or even years from the time the condom was lubricated and packaged-without drying out. For example, the carrageenan gel described in U.S. Patent 8,367,098 to Maguire has a viscosity of has a viscosity of between 30,000 and 40,000 cP, and is designed to be applied directly from the container to potentially affected skin or epithelial tissue immediately prior to sexual activity. Over the extended time periods that pre-lubricated condoms are often stored before use, carrageenan compositions such as that described in the Maguire patent would likely lose its lubricity and become sticky, rendering the condom unusable during sexual activity.

While particular embodiments of the invention have been described, the invention can be further modified within the spirit and scope of this disclosure. Those skilled in the art will recognize, or be able to ascertain using no more than routine experimentation, numerous equivalents to the specific procedures, embodiments, claims, and examples described herein. As such, such equivalents are considered to be within the scope of the invention, and this application is therefore intended to cover any variations, uses or adaptations of the invention using its general principles. Further, the invention is intended to cover such departures from the present disclosure as come within known or customary practice in the art to which this invention pertains and which fall within the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

The contents of all references, patents, and patent applications mentioned in this specification are hereby incorporated by reference and shall not be construed as an admission that such reference is available as prior art to the present invention. All of the incorporated publications and patent applications in this specification are indicative of the level of ordinary skill in the art to which this invention pertains and are incorporated to the same extent as if each individual publication or patent application was specifically indicated and individually indicated by reference.

The invention is further illustrated by the following working and prophetic examples, neither of which should be construed as limiting the invention. Additionally, to the extent that section headings are used, they should not be construed as necessarily limiting. Any use of the past tense to describe an example otherwise indicated as constructive or prophetic is not intended to reflect that the constructive or prophetic example has actually been carried out.

### EXAMPLES

The following working and prophetic examples illustrate the embodiments of the invention that are presently best known. However, it is to be understood that the following are only exemplary or illustrative of the application of the principles of the present invention. Numerous modifications and alternative compositions, methods, and systems may be devised by those skilled in the art without departing from the spirit and scope of the present invention. Thus, while the present invention has been described above with particularity, the following examples provide further detail in connection with what are presently deemed to be the most practical and preferred embodiments of the invention.

### Example 1: Preparation of a First Antiviral Lubricous Composition

The following antiviral lubricous composition was prepared in accordance with embodiments of the present invention and the procedure below.

### First Antiviral Lubricous Composition

55.70 % (w/v) deionized water
37.40 % (w/v) propylene glycol
5.00 % (w/v) of 2.5% (w/v) Sodium saccharin solution in deionized water
1.40 % (w/v) Viscarin^{®} PC 209 carrageenan powder
0.04 % (w/v) citric acid
0.01 % (w/v) sodium hydroxide
0.95 % (w/v) GeoGard ECT preservative (Lonza Consumer Care)
0.03 % (w/v) GeoGard 111 - Sodium Dehydroacetate preservative (Lonza Consumer Care)

All ingredients were combined except the Viscarin^{®} PC 209 carrageenan powder and mixed together thoroughly. With constant mixing, the composition was then gradually heated up to 75 °C. When the composition reached 50 °C, Viscarin^{®} PC 209 carrageenan powder was gradually combined until all of the carrageenan powder was added. The composition continued to be mixed at 75 °C until all of the Viscarin^{®} PC 209 carrageenan powder was dissolved, about two hours. Upon dissolution of the carrageenan powder, mixing was ceased and the composition was cooled rapidly. Once the temperature of the composition reached 30 °C, citric acid was added to bring the pH to about 6.5, +/- 0.25.

### Example 2: Preparation of a Second Antiviral Lubricous Composition

The following antiviral lubricous composition was prepared in accordance with embodiments of the present invention and the procedure below:

### Second Antiviral Lubricous Composition

88.49 % (w/v) deionized water
4.40 % (w/v) propylene glycol
5 % (w/v) of 2.5% (w/v) Sodium saccharin solution in deionized water
1.60 % (w/v) Viscarin^{®} PC 209 carrageenan powder
0.05 % (w/v) citric acid
0.32 % (w/v) 2-phenoxyethanol
0.11 % (w/v) chlorphenesin
0.03 % (w/v) GeoGard 111 - Sodium Dehydroacetate preservative (Lonza Consumer Care)

The Viscarin^{®} PC 209 sea algae extract and propylene glycol were mixed until no aggregate particles were observed. With constant mixing, approximately 90% of the volume of the water and all of the saccharin was added to the composition. While still mixing, the composition was heated to 70 °C and allowed to mix for thirty minutes until the sea algae extract was completely dissolved. Mixing was then stopped and the composition cooled to less than 30 °C. Once cooled, mixing was continued for 15 minutes and the preservatives, citric acid, and remaining water was added to the composition.

### Example 3: Exponential Effect of Carrageenan Concentration on Composition Viscosity

In order to evaluate the effect of the carrageenan on the viscosity of antiviral lubricous compositions, several compositions were formulated at varying concentrations of the carrageenan. Each of the five formulations were based on the Antiviral Lubricous Composition of Example 1, except the concentration of the carrageenan and propylene glycol was varied in each sample. As with Example 1, the carrageenan-containing sea algae extract was Viscarin^{®} PC 209. The amount of water was also adjusted in each case to give a final volume for each sample composition of 200 mL. The concentrations of components in each sample is provided in Table 1, below.

**Table 1: Concentrations of Components Used in Viscosity Studies**

| Sample Number | Sea Algae Extract (w/v) | Propylene Glycol (w/v) |
|---|---|---|
| 1 | 1.40 | 37.4 |
| 2 | 1.73 | 24.0 |
| 3 | 2.10 | 8.8 |
| 4 | 2.15 | 6.6 |
| 5 | 2.20 | 4.4 |

Each of the viscosity measurements were taken using a Brookfield LVF Dial Reading Viscometer, using a #2 spindle at 12 revolutions per minute, according to instructions provided with the instrument. The viscosity of each sample is provided in Table 2, below.

**Table 2: Measured Viscosities**

| Sample Number | Measured Viscosity (cP) |
|---|---|
| 1 | 1975 |
| 2 | 2900 |
| 3 | 4000 |
| 4 | 4400 |
| 5 | 4750 |

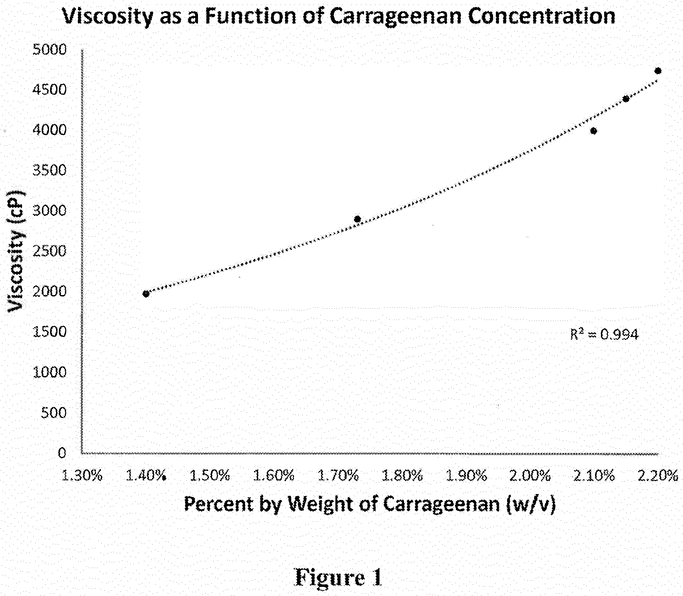

The relationship between the viscosity of the antiviral lubricous composition as a function of the carrageenan (sea algae extract) concentration is illustrated in Figure 1. Fitting the data to an exponential model using Microsoft Excel yields a trendline with an R² value of 0.994, indicating a strong correlation between the data and the model.

### Example 4: Randomized, Double-Blind Study of Cervical HPV Transmission

A randomized, double-blind, placebo-controlled, phase 2B trial was conducted to evaluate the efficacy of the lambda-carrageenan-containing antiviral lubricant composition of Example 1 in reducing genital HPV incidence and prevalence among young sexually active women. The study was conducted at the McGill and Concordia University Health Services Clinics and the CLSC Samuel-de-Champlain, three outpatient clinics that are located in the greater Montreal area, Canada. The study complied with the principles of the Declaration of Helsinki, the ICH Guideline for Good Clinical Practice, and the regulatory requirements of Natural Health Product Directorate and Health Canada.

Study participants were women aged 18 years and older that were recruited through advertisements mainly displayed in colleges and universities in Montreal and on social media. Participants were eligible if they (i) planned to remain in Montreal for at least the next year, (ii) had vaginal sex with a male partner in the last three months, (iii) were expecting to have vaginal intercourse in the next three months with the same or different male partner(s), and (iv) were not currently in a relationship for longer than six months. Other inclusion criteria were: having an intact uterus, being HIV negative, using a medically acceptable method of contraception and intending to continue using it for the duration of the study. Exclusion criteria were: having a history of cervical lesions, cancer or genital warts, being pregnant or currently-breast feeding, having had a pregnancy, an abortion or a genital surgery in the last 6 weeks and having a known allergy or hypersensitivity to any vaginal lubricant or component(s) within the antiviral lubricant composition of Example 1.

Participants were randomly assigned in a 1:1 ratio to either the carrageenan-based antiviral lubricant composition of Example 1 or a placebo lubricant that didn't contain carrageenan, using a computer-generated concealed sequence with a block size of eight. Both lubricants were clear, odorless, tasteless, of similar viscosity, and were contained in identical bottles on which the different product codes were inscribed. Randomization and product code assignment was done via an online administration platform upon submission of the participant's basic identification information. Participants were provided with lubricant supplies corresponding to their assigned product code through the duration of the study. Participants, nurses, laboratory personnel and investigators were masked to group allocation. Only people involved in data analysis were unblinded at the time the database was locked for interim analysis.

Participants were asked to self-apply the assigned lubricous composition every other day for the first month (irrespective of whether or not they were engaging in sexual activities) and prior to and following each intercourse during the entire study period. They were instructed to apply the lubricant (at least 10 mL at each application) inside their vagina and on their genital area using their fingers. At each visit, participants were provided with sufficient lubricant supplies to cover the between-visit periods. Participants could also request additional bottles of lubricant at any time during the study.

The study period was 1 year, and assessments were performed at seven different time points during follow-up. Clinical visits were scheduled at enrollment, 2 weeks, and at 1, 3, 6, 9, and 12 months. At each visit, the participant met with a research nurse, completed a questionnaire in a private room and provided a vaginal specimen for HPV testing. At the first visit, a pregnancy test and an HIV test were performed to assess eligibility before randomization. The pregnancy test was done using the QuickStep Plus hCG Combo Test Kit with a urine sample. The HIV test was done using the INSTI HIV-1 Rapid Antibody Test with a capillary blood sample. Participants with a positive result at either of these tests were immediately referred to a physician.

Computer-assisted self-administered questionnaires were used to collect data on sociodemographic and behavioral variables, as well as on sexual activity and HPV vaccination history. HPV testing was performed on self-collected vaginal samples. Self-collection methods have been validated for research and clinical uses and acceptable for women (*see* Durant, L.E., and Carey, M.P., (2000) Archives of Sexual Behavior 29 (4):309-322 and Gravitt, P.E., et al., (2001) Cancer epidemiology, biomarkers & prevention: a publication of the American Association for Cancer Research, cosponsored by the American Society of Preventive Oncology 10 (2):95-100.). Participants were instructed to gently insert a Dacron^{™} swab into their vagina at least 5 cm, until it could not physically go any further, and then to rotate the swab inside their vagina for three full rotations.

HPV detection and genotyping to determine the incidence of an HPV infection not present at the baseline checkup were performed with the PGMY polymerase chain reaction (PCR) protocol coupled with a Linear Array assay, provided by Roche Holding AG, which allows the detection and typing of 36 genital HPV types (6, 11, 16, 18, 26, 31, 33, 34, 35. 39, 40, 42, 44, 45, 51, 52, 53, 54, 56, 58, 59, 61, 62, 66, 67, 68, 69, 70, 71, 72, 73, 81, 82, 83, 84, 89). Types were analyzed separately, although they were divided into three subgenera based on tissue tropism and oncogenicity- Alphapapillomavirus subgenera 1, 2 and 3. Subgenus 1 includes species of HPV types of low oncogenic risk that cause benign lesions such as warts (HPVs 6, 11, 40, 42, 44, 54); subgenus 2 includes species of HPV types of high oncogenic risk that cause high-grade and cancerous lesions (HPVs 16, 18, 26, 31, 33, 34, 35, 39, 45, 51, 52, 53, 56, 58, 59, 66, 67, 68, 69, 70, 73, 82); and subgenus 3 includes species of mostly commensal HPV types that do not cause any clinical lesion (HPVs 61, 62, 71, 72, 81, 83, 84, 89). Participants were asked to abstain from oral sex, vaginal intercourse and use of sample lubricant 48 hours prior to a scheduled visit. It is not believed that carrageenan interferes with HPV detection and genotyping.

Compliance with study instructions was assessed at each visit with self-assisted questionnaires. Participants were asked to log on to a secure website and complete an electronic calendar on their daily sexual activities, condom use and adherence to the intervention. To minimize recall bias, participants were instructed to update their information for any given day within seven days, after which they were unable to view or modify the information for that day. All lubricant bottles given to participants were entered in their records. Participants were asked to bring back their bottles at each visit, regardless of whether they were empty or full, so the research nurse could weigh them and estimate the amount of lubricant used. Participants were told that they were not evaluated on or compensated for the amount of lubricant use. Additionally, lubricant safety was assessed at each visit during the meeting with the research nurse. Participants had the opportunity to report any adverse events at any time using the electronic calendar. Research nurses were automatically notified via email each time a participant reported an adverse event and an appointment with the study physician (PPT) was scheduled if needed. The Female Genital Grading Table for Use in Microbicide Studies from the Division of AIDS Table for Grading the Severity of Adult and Pediatric Adverse Events (version 1.0, November 2007) was used for the reporting and grading of adverse events. A pap test was performed for participants exiting the study who were 25 years and older and had not received an exam in the past three years.

It was calculated that a total of 463 participants were required to detect a 50% difference in incidence with 80% power using a two-sided significance level of 0.05. The total sample size needed to detect a 50% difference in clearance with the same parameters was 388. Losses to follow-up of about 10% at 12 months was assumed, so the study aimed to recruit at least 500 participants. An interim analysis was performed when the study reached half of the target same size.

In the interim analysis, only the incidence of new HPV infections was determined. Analyses were done by intention-to-treat. Hazard ratios (HR) and 95% confidence intervals (CI) were estimated using univariate Cox models considering the first occurrence of a new infection in each participant. Four different definitions of the outcome were considered: an infection by any HPV type and an infection by types belonging to the three Alphapapillomavirus subgenera defined above.

Two different models were computed taking into account correlated data and considering all new episodes of HPV types by each participant through the study period. The first model was a Cox model stratified by HPV types and clustered by participants. The second model was a mixed effect survival-time model considering an exponential distribution and a random effect for participants. All statistical analyses were performed using Stata version 14.2.

Planned sub-group analyses were performed for the main outcome definition based on the following baseline characteristics: age, ethnicity, marital status, smoking status, age at first intercourse, number of lifetime sexual partners, number of sexual partners in the month before enrollment, HPV status and vaccination status. A planned sub-group analysis was also done based on cumulative compliance to lubricant use before intercourse at the time of failure or censoring. A post hoc sensitivity analysis was conducted on participants who validated their vaccination status after we solicited a confirmation by email.

### Data

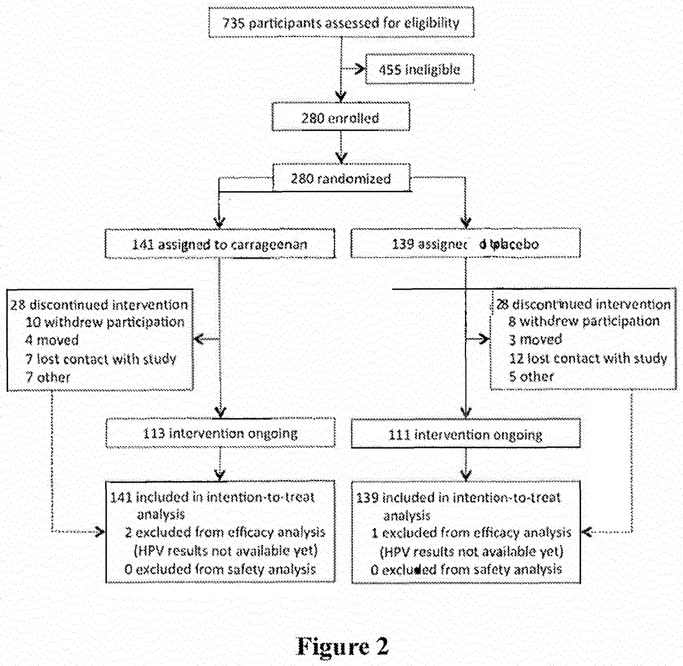

Figure 2 shows the trial profile. Between January 16, 2013 and June 9, 2017, 735 women were recruited and assessed for eligibility. Of these, 275 didn't meet the inclusion criteria, 101 declined to participate and 79 were deemed ineligible for other reasons. Finally, 280 women were randomly assigned to either the carrageenan (n=141) or the placebo (n=139) lubricant. All participants received the allocated intervention. 28 (20%) participants in the carrageenan arm and 28 (20%) participants in the placebo arm withdrew from the study before completing the seven follow-up visits. All participants were included in the safety analysis. Only 3 participants were excluded from efficacy analysis: 2 in the carrageenan arm and 1 in the placebo arm, because their HPV results were not available at the time the database was locked for interim analysis.

As shown in Table 3, below, participants' baseline and follow-up characteristics were similar between the two arms. Participants were from various ethnic backgrounds and most were single. The mean age was 24.0 years (SD: 5.3). 147 (52.5%) participants were HPV-positive at baseline: 67 (47.5%) in the carrageenan arm and 80 (57.6%) in the placebo arm. More individuals were infected by more than one HPV type (co-infection) in the placebo arm (n=56, 40.3%) than in the carrageenan arm (n=42, 29.8%). Overall, the between-arm differences in positivity for single and multiple type infections at baseline did not reach statistical significance (p=0.28). Frequencies for vaccination status are based on the self-reporting at enrollment, as well as after validation by email confirmation. Overall, 117 (41.8%) participants reported having been vaccinated: 80 (68.4%) with the quadrivalent vaccine, 2 (1.7%) with a mix of the quadrivalent and the nonavalent vaccines, 0 (0%) with the bivalent vaccine, and 35 (29.9%) could not remember which vaccine they received. 99 (35%) participants confirmed their vaccination status by email (table 1). 26 of the 31 participants (84%) who confirmed that they had been vaccinated were able to provide either a proof of vaccination (11 participants, 35%), or at least additional details to support that they had been vaccinated (15 participants, 48%). 15 of the 99 participants who replied to confirmation emails changed the answer they gave in the enrollment questionnaire.

**Table 3: Baseline and follow-up characteristics of participants**

| | | **Carrageenan (n=141)** | **Placebo (n=139)** |
|---|---|---|---|
| **Age - years** | | | |
| | Mean (SD) | 24.6 (5.8) | 23.4 (4.7) |
| | Median (IQR) | 23.0 (20.4-27.0) | 22.2 (19.9-25.2) |
| | Missing, n (%) | 0 (0.0) | 0 (0.0) |

| **Ethnicity - n** (%) | | | |
|---|---|---|---|
| | French Canadian | 29 (20.6%) | 33 (23.7%) |
| | English Canadian | 38 (27.0%) | 43 (30.9%) |
| | Black Canadian | 11 (7.8%) | 9 (6.5%) |
| | Latin American | 13 (9.2%) | 13 (9.4%) |
| | East Asian | 9 (6.4%) | 14 (10.1%) |
| | Other | 37 (26.2%) | 25 (18.0%) |
| | Missing | 4 (2.8%) | 2 (1.4%) |

| **Marital status - n (%)** | | | |
|---|---|---|---|
| | Single | 91 (64.5%) | 97 (69.8%) |
| | Living with a partner | 12 (8.5%) | 14 (10.1%) |
| | Married | 3 (2.1%) | 1 (0.7%) |
| | Divorced/separated/widowed | 35 (24.8%) | 27 (19.4%) |
| | Missing | 0 (0.0%) | 1 (0.0%) |

| **Smoking status - n (%)** | | | |
|---|---|---|---|
| | Never | 96 (68.1%) | 78 (56.1%) |
| | Past | 33 (23.4%) | 40 (28.8%) |
| | Current | 12 (8.5%) | 20 (14.4%) |
| | Missing | 0 (1.1%) | 1 (0.7%) |

| **Age at first intercourse - years** | | | |
|---|---|---|---|
| | Mean (SD) | 17.5 (3.1) | 16.9 (2.5) |
| | Median (IQR) | 17 (16-19) | 17 (16-18) |
| | Missing, n (%) | 3 (2.1%) | 3 (2.2%) |

| **Lifetime sex partners - n** (%) | | | |
|---|---|---|---|
| | 1-3 | 32 (22.7%) | 30 (21.6%) |
| | 4-6 | 32 (22.7%) | 19 (13.7%) |
| | 7-10 | 26 (18.4%) | 32 (23.0%) |
| | 11-20 | 25 (17.7%) | 31 (22.3%) |
| | > 20 | 26 (18.4%) | 27 (19.4%) |
| | Missing | 0 (0.0%) | 0 (0.0%) |

| **Sex partners in the last month - n (%)** | | | |
|---|---|---|---|
| | 0 | 29 (20.6%) | 24 (17.3%) |
| | 1 | 87 (61.7%) | 85 (61.2%) |
| | ≥ 2 | 25 (17.7%) | 30 (21.6%) |
| | Missing | 0 (0.0%) | 1 (0.0%) |

| **HPV DNA status - n (%)** | | | |
|---|---|---|---|
| | Negative | 72 (51.1%) | 58 (41.7%) |
| | Mono-infection | 25 (17.7%) | 24 (17.3%) |
| | Co-infection | 42 (29.8%) | 56 (40.3%) |
| | Missing PCR results^{a} | 2 (1.4%) | 1 (1.7%) |
| | Subgenus 1^{b} | 16 (11.4%) | 29 (20.9%) |
| | Subgenus 2^{c} | 57 (40.4%) | 64 (46.0%) |
| | Subgenus 3^{d} | 29 (20.6%) | 45 (32.4%) |

| **Vaccination status - n (%)** | | | |
|---|---|---|---|
| | Yes | 54 (38.9%) | 63 (45.7%) |
| | No | 80 (57.6%) | 74 (53.6%) |

| | | **Carrageenan (n=141)** | **Placebo (n=139)** |
|---|---|---|---|
| | Missing | 5 (3.6%) | 1 (0.7%) |

| **Validated vaccination status - n (%)** | | | |
|---|---|---|---|
| | Yes | 15 (10.8%) | 16 (11.6%) |
| | No | 35 (25.2%) | 33 (23.9%) |
| | Missing | 89 (64.0) | 89 (64.5%) |

| **Follow-up time - months** | | | |
|---|---|---|---|
| | Mean (SD) | 8.5 (6.0) | 8.4 (5.8) |
| | Median (IQR) | 8.8 (2.6-13.7) | 9.8 (2.6-13.0) |

| **Number of visits/woman - n** | | | |
|---|---|---|---|
| | Mean (SD) | 4.9 (2.0) | 5.1 (1.9) |
| | Median (IQR) | 5 (3-7) | 6 (3-7) |

| | | | |
|---|---|---|---|
| ^{a} Missing results correspond to vaginal samples that were collected but not yet analyzed by the laboratory; ^{b} Subgenus 1 group includes HPVs 6, 11, 40, 42, 44, and 54; ^{c} Subgenus 2 group includes HPVs 16, 18, 26, 31, 33, 34, 35, 39, 45, 51, 52, 53, 56, 58, 59, 66, 67, 68, 69, 70, 73, and 82; ^{d} Subgenus 3 group includes HPVs 61, 62, 71, 72, 81, 83, 84, and 89. | | | |

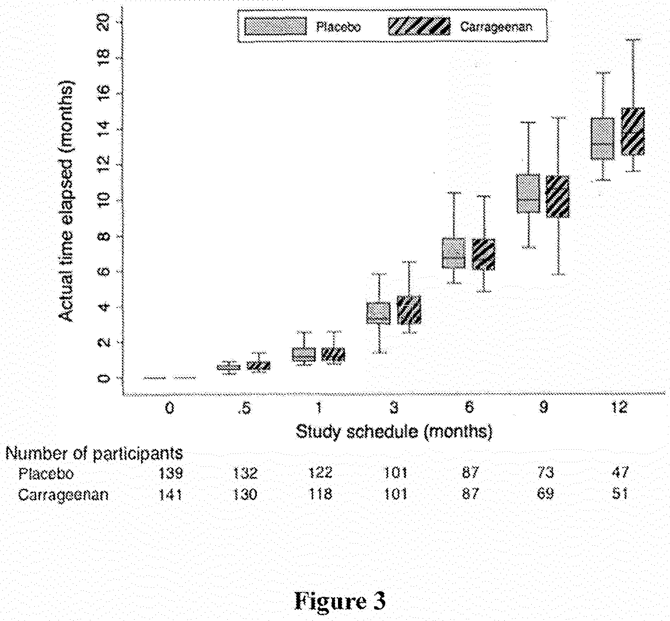

The median follow-up time for the whole cohort was 9.2 months (IQR: 2.6-13.2). The follow-up time and number of visits per woman were comparable between the two arms (Table 3). Figure 3 shows the actual time elapsed between enrollment and subsequent follow-up visits relative to the planned schedule, as well as the number of participants who completed each visit. There were no significant differences between the two arms with respect to time elapsed between visits. At the interim analysis closing date, 98 (35%) participants completed all seven visits: 51 (36%) in the carrageenan arm and 47 (34%) in the placebo arm.

Table 4, shown below, shows the visit- and arm-specific prevalence of HPV infection for the four types included in the quadrivalent vaccine and for the three Alphapapillomavirus subgenera. The carcinogenic HPV types (subgenus 2) were the most prevalent among the study participants. Except for HPV16, prevalence of the quadrivalent vaccine-targeted HPV types was low. Missing results correspond to vaginal samples that were collected but had not yet been tested at the analysis closing date.

**Table 4: HPV prevalence [n, (%)] by visit by arm**

| | | **Visit 1** | **Visit 2** | **Visit 3** | **Visit 4** | **Visit 5** | **Visit 6** | **Visit 7** |
|---|---|---|---|---|---|---|---|---|
| **Carrageenan arm** | | **(n=141)** | **(n=130)** | **(n=118)** | **(n=101)** | **(n=87)** | **(n=69)** | **(n=51)** |
| | Any HPV | 67 (47.5%) | 58 (44.6%) | 57 (48.3%) | 50 (49.5%) | 42 (48.3%) | 36 (52.2%) | 24 (47.1%) |
| | HPV6 | 3 (2.1%) | 2 (1.5%) | 3 (2.5%) | 3 (3.0%) | 2 (2.3%) | 2 (2.9%) | 2 (3.9%) |
| | HPV11 | 1 (0.7%) | 1 (0.8%) | 1 (0.9%) | 2 (2.0%) | 0 (0.0%) | 0 (1.1%) | 0 (0.0%) |
| | HPV16 | 6 (4.3%) | 9 (6.9%) | 9 (7.6%) | 8 (7.9%) | 5 (5.8%) | 3 (4.4%) | 2 (3.9%) |
| | HPV18 | 3 (2.1%) | 2 (1.5%) | 2 (1.7%) | 1 (1.0%) | 1 (1.2%) | 1 (1.5%) | 1 (2.1%) |
| | Subgenus 1^{a} | 16 (11.4%) | 13 (10.0%) | 14 (11.9%) | 14 (13.9%) | 12 (13.8%) | 11 (15.9%) | 9 (17.7%) |
| | Subgenus 2^{b} | 57 (40.4%) | 49 (37.7%) | 46 (39.0%) | 38 (37.6%) | 31 (35.6%) | 30 (43.5%) | 20 (39.2%) |
| | Subgenus 3^{c} | 29 (20.6%) | 25 (19.2%) | 25 (21.2%) | 25 (24.8%) | 18 (20.7%) | 15 (21.7%) | 13 (25.5%) |
| | Missing^{d} | 2 (1.4%) | 2 (1.5%) | 2 (1.7%) | 4 (4.0%) | 4 (4.6%) | 3 (4.4%) | 2 (3.9%) |

| **Placebo arm** | | **(n=139)** | **(n=132)** | **(n=122)** | **(n=101)** | **(n=87)** | **(n=73)** | **(n=47)** |
|---|---|---|---|---|---|---|---|---|
| | Any HPV | 80 (57.6%) | 85 (64.4%) | 75 (61.5%) | 59 (58.4%) | 49 (56.3%) | 37 (50.7%) | 22 (46.8%) |
| | HPV6 | 2 (1.4%) | 2 (1.5%) | 2 (1.6%) | 1 (1.0%) | 1 (1.2%) | 1 (1.4%) | 1 (2.1%) |
| | HPV11 | 0 (0.0%) | 0 (0.0%) | 0 (0.0%) | 1 (1.0%) | 1 (1.2%) | 1 (1.4%) | 1 (2.1%) |
| | HPV16 | 5 (3.6%) | 5 (3.8%) | 5 (4.1%) | 4 (4.0%) | 5 (5.8%) | 3 (4.1%) | 1 (2.1%) |
| | HPV18 | 3 (2.2%) | 3 (2.3%) | 3 (2.5%) | 3 (3.0%) | 1 (1.2%) | 2 (2.7%) | 1 (2.1%) |
| | Subgenus 1^{a} | 29 (20.9%) | 31 23.5%) | 30 (24.6%) | 24 (23.8%) | 19 (21.8%) | 17 (23.3%) | 12 (25.5%) |
| | Subgenus 2^{b} | 64 (46.0%) | 68 (51.5%) | 60 (49.2%) | 44 (43.6%) | 38 (43.7%) | 30 (41.1%) | 15 (31.9%) |
| | Subgenus 3^{c} | 45 (32.4%) | 47 (35.6%) | 40 (32.8%) | 31 (30.7%) | 30 (34.5%) | 22 (30.1%) | 18 (38.3%) |
| | Missing^{d} | 1 (0.7%) | 2 (1.5%) | 3 (2.5%) | 4 (4.0%) | 4 (4.6%) | 6 (8.2%) | 1 (2.1%) |

| | | | | | | | | |
|---|---|---|---|---|---|---|---|---|
| ^{a} Subgenus 1 group includes HPVs 6, 11, 40, 42, 44, and 54; ^{b} Subgenus 2 group includes HPVs 16, 18, 26, 31, 33, 34, 35, 39, 45, 51, 52, 53, 56, 58, 59, 66, 67, 68, 69, 70, 73, and 82; ^{c} Subgenus 3 group includes HPVs 61, 62, 71, 72, 81, 83, 84, and 89; ^{d} Missing results correspond to vaginal samples that were collected but not yet analyzed by the laboratory. | | | | | | | | |

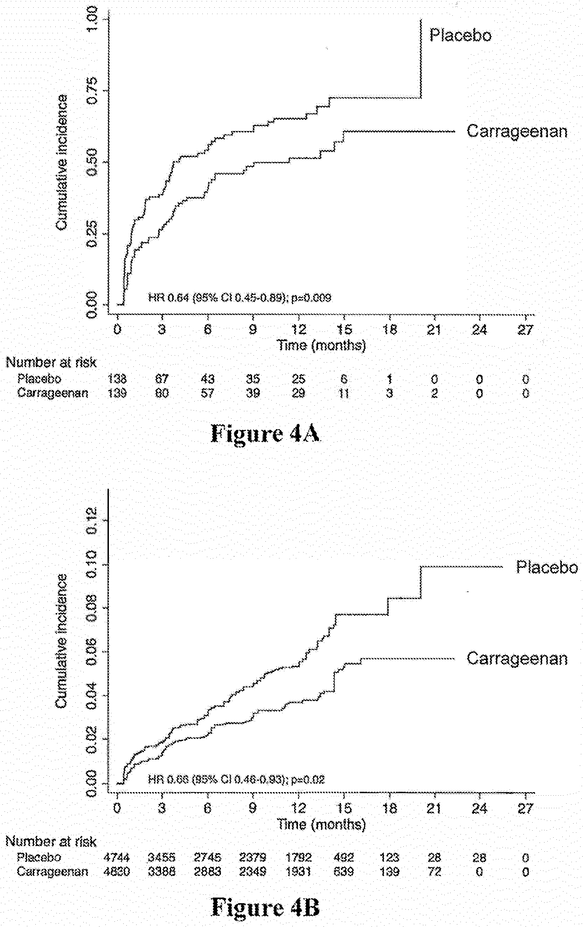

Figure 4A and Figure 4B show the cumulative incidence of a newly detected HPV infection (primary outcome), for both arms. 137 participants acquired at least one HPV type: 59 (42%) in the carrageenan arm and 78 (56%) in the placebo arm. The HR for the first occurrence of a new infection was 0.64 (95%CI: 0.45-0.89, p=0.009) (Figure 4A). When considering all new HPV types acquired by each participant (not only the first infection), 139 infections occurred in the carrageenan arm versus 198 in the placebo arm (rate ratio = 0.68, 95%CI: 0.55-0.85, p=0.0007). Figure 4B shows the cumulative incidence of all new types by arm taking into account the multiplicity of HPV types that subjects could have acquired at each visit. Two models were computed to accommodate the correlated data structure and multiplicity of HPV types to which participants were exposed. The HR for the first model, a Cox model stratified by HPV types and clustered by participants, was 0.66 (95%CI: 0.46-0.93, p=0.02). The second model, a mixed-effects parametric survival-time model, yielded a HR of 0.60 (95%CI: 0.39-0.94, p=0.03).

Table 5, below, shows incident cases, overall and by subgenera, for both arms with two estimates of mean time to the first infection with a new HPV type: the actuarial calculation that took into account censoring and an arithmetic calculation, restricted to complete observations, i.e., those who had a documented episode of a new type. The actuarial mean time to a first infection was significantly greater in the carrageenan arm than in the placebo arm. When restricted to those who acquired a first episode the differences in time to acquisition were small and non-significant. The reduction in incidence of new HPV infections was significant for subgenus 1 (HR=0.47, 95%CI: 0.24-0.89) and subgenus 2 types (HR=0.64, 95%CI: 0.44-0.94).

**Table 5: Incidence of and average time to a new HPV infection by arm**

| | **Carrageenan** | | | | **Placebo** | | | | **Comparison** | |
|---|---|---|---|---|---|---|---|---|---|---|
| | **Incident cases** | **Actuarial mean (95% CI)** | **Arithmetic mean (95% CI)** | **Median (95% CI)** | **Incident cases** | **Actuarial mean (95% CI)** | **Arithmetic mean (95% CI)** | **Median (95% CI)** | **Hazard Ratio (95% CI)** | **p-value** |
| **Any HPV type** | 59 | 11.8^{d} (10.0-13.7) | 3.5 (2.6-4.5) | 11.3 (6.0- )^{e} | 78 | 8.3 (6.8-9.9) | 3.1 (2.3-4.0) | 3.7 (3.2-7.1) | 0.64 (0.45-0.89) | 0.009 |
| **Subgenus 1^{a}** | 14 | 19.7^{d} (18.4-21.0) | 5.1 (2.5-7.7) | NR | 27 | 15.3^{d} (13.7-16.8) | 4.8 (3.0-6.6) | NR | 0.47 (0.24-0.89) | 0.02 |
| **Subgenus 2^{b}** | 47 | 13.8⁴ (12.0-15.7) | 4.0 (2.8-5.2) | 14.9 (9.3- )^{e} | 64 | 10.4 (8.8-12.0) | 3.7 (2.6-4.7) | 9.0 (5.8-14.0) | 0.64 (0.44-0.94) | 0.02 |
| **Subgenus 3^{c}** | 29 | 17**.**0^{d} (15.3-18.6) | 4.2 (2.7-5.8) | NR | 39 | 13.7^{d} (12.1-15.2) | 4.8 (3.4-6.2) | NR | 0.69 (0.42-1.11) | 0.15 |

| | | | | | | | | | | |
|---|---|---|---|---|---|---|---|---|---|---|
| ^{a} Subgenus 1 group includes HPVs 6, 11, 40, 42, 44, and 54; ^{b} Subgenus 2 group includes HPVs 16, 18, 26, 31, 33, 34, 35, 39, 45, 51, 52, 53, 56, 58, 59, 66, 67, 68, 69, 70, 73, and 82; ^{c} Subgenus 3 group includes HPVs 61, 62, 71, 72, 81, 83, 84, and 89. ^{d} The mean is underestimated as the largest observed analysis time is censored. ^{e} The upper confidence limit could not be determined because the survival function never falls below 0.5. NR=Not reached | | | | | | | | | | |

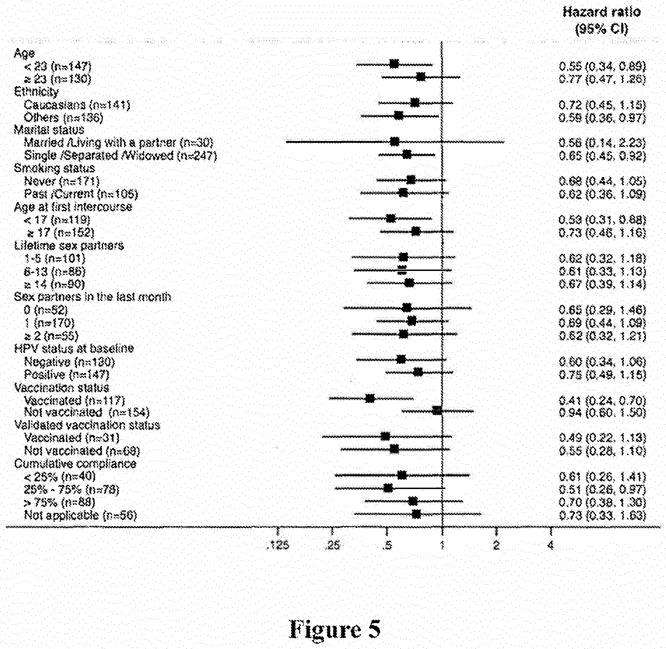

Sub-group analyses were done considering only the first occurrence of a newly detected HPV infection (Figure 5). The results favoring the intervention were consistent across all sub-groups except for self-reported vaccination. There was no indication of heterogeneity of effects when stratified by validated vaccination status. There was no indication of a dose-response relationship with the estimated cumulative compliance.

Table 6, below, shows the 258 adverse events reported by 105 (37.5%) participants using the daily electronic calendar. There were more adverse events in the carrageenan (150 reported by 62 (43.9%) participants) than in the placebo arm (108 reported by 43 (30.9%) participants). However, none of these adverse events was judged to be related to the study lubricants by the study physician (PPT) who was blinded to group allocation. No participant withdrew or was withdrawn from the study because of an adverse event.

**Table 6: Adverse events**

| | | | | | | |
|---|---|---|---|---|---|---|
| | | **Mild** | **Moderate** | **Severe** | **Not graded** | **Total** |

| **Carrageenan arm** | | | | | | |
|---|---|---|---|---|---|---|
| | Any | 62 (27) | 17 (15) | 7 (7) | 64 (25) | 150 (62) |
| | Unusually painful or heavy period | - | - | - | - | - |
| | Vaginal bleeding in between menstrual periods | 11 (3) | - | - | - | 11 (3) |
| | Pain during vaginal sex | 3 (2) | 5 (4) | 1 (1) | 5 (3) | 14 (8) |
| | Unusual vaginal discharge | 14 (4) | 4 (4) | - | 6 (1) | 24 (8) |
| | Itching, burning or pain in the genital area | 28 (12) | 5 (4) | 3 (3) | 10 (4) | 46 (16) |
| | Genital sore/ulcer | 1 (1) | - | - | - | 1 (1) |
| | Needing to urinate more often than usual | 1 (1) | 1 (1) | - | - | 2 (1) |
| | Pain while urinating | 1 (1) | - | 2 (2) | 5 (1) | 8 (4) |
| | Blood in urine | 1 (1) | - | 1 (1) | 3 (1) | 5 (3) |
| | Lower abdominal pain | 2 (2) | - | - | - | 2 (2) |
| | Lower back pain | - | - | - | - | - |
| | Other | - | 2 (2) | - | 35 (15) | 37 (16) |

| **Placebo arm** | | | | | | |
|---|---|---|---|---|---|---|
| | Any | 31 (16) | 32 (17) | 10 (8) | 35 (22) | 108 (43) |
| | Unusually painful or heavy period | 1 (1) | 6 (1) | 1 (1) | 1 (1) | 9 (3) |
| | Vaginal bleeding in between menstrual periods | 6 (3) | 1 (1) | - | - | 7 (4) |
| | Pain during vaginal sex | - | 1 (1) | 1 (1) | - | 2 (2) |
| | Unusual vaginal discharge | 9 (2) | 5 (2) | 1 (1) | - | 15 (2) |
| | Itching, burning or pain in the genital area | 12 (7) | 11 (7) | 6 (4) | 3 (3) | 32 (11) |
| | Genital sore/ulcer | 2 (2) | 4 (1) | - | - | 6 (2) |
| | Needing to urinate more often than usual | - | 1 (1) | - | 4 (2) | 5 (3) |
| | Pain while urinating | - | - | - | 3 (3) | 3 (1) |
| | Blood in urine | - | - | - | 1 (1) | 1 (1) |
| | Lower abdominal pain | - | - | - | - | - |
| | Lower back pain | - | - | - | - | - |
| | Other | 1 (1) | 3 (3) | 1 (1) | 23 (12) | 28 (14) |

| | | | | | | |
|---|---|---|---|---|---|---|
| Data are number of adverse events reported (number of participants affected). Some participants had more than one event. | | | | | | |

### Study Results and Conclusions

In this analysis, the use of the carrageenan-based lubricant prior to and following each intercourse was associated with a 36% protective effect when compared to the use of a placebo lubricant. A protective effect was observed for both low-risk and high-risk HPV types. The carrageenan-based lubricant appeared to be safe and well tolerated. Although carrageenan concentrations decreased with time, inhibition remained high (>80% in most samples) even 8h after lubricant application.

The carrageenan-based lubricants showed activity against all mucosotropic HPV types infecting the anogenital tract irrespective of taxonomic grouping. Although by design CATCH study participants were at high risk of being exposed to HPV, the actual proportion of these participants who were exposed to new (not present at baseline) HPV types during follow-up (and to how many and to which types they were exposed) is not known. Nevertheless, owing to randomization, overall exposure to HPV can be assumed to be comparable between groups. However, there may have been a difference in the risk of being infected by a new HPV type, since there were more co-infections at baseline in the placebo group (40%) than in the carrageenan group (30%), although this difference was not statistically significant.

Additionally, the number of adverse events reported by the participants was surprisingly high. However, the study physician (PPT), who was masked to group allocation, did not attribute any of them to the use of the study lubricants. Moreover, no participant withdrew from the study because of an adverse event. Participants had the opportunity to report adverse events on a daily basis through the electronic calendar. Considering a mean follow-up of 257 days, the 280 participants had a total of 71,960 opportunities to report an adverse event. This arguably high number of opportunities to report any noticeable change could explain, in part, the high number of adverse events observed. However, the adverse events reported were mostly mild, not attributed to lubricant use, and comparable between groups. Consequently, the interim analysis suggests that the use of the carrageenan-based lubricant can reduce the risk of new genital HPV infections in women.

### Example 5: Characterization of the disaccharide content of Antiviral Lubricous Compositions

Iota-carrageenan comprises alternating disaccharides of D-galactaose-4-sulfate and 2-sulfo-3,6-anhydro-D-galactose. Kappa-carrageenan comprises alternating disaccharides of D-galactose-4-sulfate and 3,6-anyhdro-D-galactose. Lambda-carrageenan comprises alternating disaccharides of D-galactose-2-sulfate (1-3 linked) and D-galactose-2,6-disulfate (1,4 linked). As a result, lambda-carrageenan typically contains more sulfate groups per polymer and substantially fewer anhydrogalactose residues, which are commonly found in iota-carrageenan and kappa-carrageenan. Consequently, the relative ratio of lambda- to iota- to kappa-carrageenan in an antiviral lubricous composition can be determined by several analytical techniques, including but not limited to infrared spectroscopy (IR), nuclear magnetic resonance (NMR) spectroscopy (*see* de Araujo, C.A., et al., (2013) Carbohydrate Polymers 91:483-491, and liquid chromatography coupled to mass spectroscopy (LC-MS) *(see* Diez, F., et al., (2017) "Development of an Analytical Method to Determine the amount of ê Carrageenan in HPMC Capsules by LCMS," American Association of Pharmaceutical Scientists Poster Submission, obtained from the internet at http://abstracts.aaps.org/Verify/AAPS2017/PosterSubmissions/M8109.pdf on May 3, 2018).

In particular, IR spectrum of antiviral lubricous composition can be utilized to determine both a profile of the composition that can be compared against other lambda-carrageenan-containing compositions, as well as a relative molar ratio of the lambda-, iota-, and kappa-carrageenan forms within the composition *(see* Volery, P., et al., (2004) J. Agric. Food Chem. 52 (25):7457-7463). Within the fingerprint region of a typical IR spectrum (between about 800 cm⁻¹ and about 1250 cm⁻¹), carrageenan has several strong, broad absorption bands for residues or functional groups commonly found within each polymer, as well as a strong absorption maximum between about 1065 cm⁻¹ and about 1020 cm⁻¹, particularly about 1050 cm⁻¹. The intensity of the absorbance of a particular band corresponding to a residue or functional group, relative to the intensity of the major absorption band at 1050 cm⁻¹, can be used to determine the relative abundance of a particular form of carrageenan with the IR sample. Characteristic absorption bands and their intensities relative to the major absorption band at 1050 cm⁻¹ are illustrated below in Table 7.

**Table 7: Common IR absorption bands in carrageenan**

| Wave Number (cm⁻¹) | Molecular Assignment | Absorbance relative to 1050 cm⁻¹ | | |
|---|---|---|---|---|
| | | Kappa | Iota | Lambda |
| 1220-1260 | Ester sulfate | 0.2-1.2 | 1.2-1.6 | 1.4-2.0 |
| 928-933 | 3,6-anydrogalactose | 0.2-0.6 | 0.2-0.4 | 0-0.2 |
| 840-850 | Galactose-4-sulfate | 0.1-0.5 | 0.2-0.4 | - |
| 825-830 | Galactose-2-sulfate | - | - | 0.2-0.4 |
| 810-820 | Galactose-6-sulfate | - | - | 0.1-0.3 |
| 800-805 | 3,6-anhydrogalactose-2-sulfate | 0-0.2 | 0.2-0.4 | - |

A study is conducted in accordance with principles of the present invention to characterize the carrageenan within a sample of the antiviral lubricous composition. A sample of the antiviral lubricous composition is subjected to IR spectroscopic analysis according to the procedure described in the Food and Agricultural Organization of the United Nations/World Health Organization joint compendium of food additive specifications (*see* "Carrageenan, Compendium of Food Additive Specifications FAO JEFCA Monographs 16; FAO/WHO Publications; Rome, Italy; pp. 7-12). It is expected that antiviral lubricous compositions of the present invention possess a unique and characteristic IR spectrum that can be compared against other compositions that contain lambda-carrageenan. It is also expected that the IR spectrum for the antiviral lubricous composition possesses peaks between 810 cm⁻¹ and 820 cm⁻¹ as well as 825 cm⁻¹ and 830 cm⁻¹, indicating the presence of lambda-carrageenan within the antiviral lubricous composition. It is further expected that the molar ratio of lambda-carrageenan in the antiviral lubricous composition is greater relative to the molar ratio of kappa-carrageenan and iota-carrageenan.

**Alternative expressions of the inventive concept are set out in the following clauses:**
1. A method for reducing, inhibiting, or ameliorating the transmission of HPV between partners engaging in sexual activity, the steps comprising:
   (A) providing a pourable, homogeneous antiviral lubricous composition comprising:
      (i) a water-soluble sea algae extract comprising lambda-carrageenan, wherein
         (a) lambda-carrageenan comprises at least 50% by weight of the sea algae extract, including at least about 60, 70, 80, or 85 percent by weight, up to at least about 90% by weight of the sea algae extract, particularly between about 80% by weight and about 90% by weight of the sea algae extract, and
         (b) the sea algae extract comprises at least about 0.001% by weight of the antiviral lubricous composition, including at least about 0.01, 0.1, 1, or 3% by weight, up to at least about 5% by weight of the antiviral lubricous composition, particularly between about 1% by weight and about 3% by weight of the antiviral lubricous composition, and
      (ii) a pH-adjusting agent comprising an acid, particularly an organic acid, and more particularly citric acid,
      wherein the antiviral lubricous composition has a viscosity of less than about 10,000 cP, particularly between about 500 cP and about 8,000 cP, more particularly between about 1,000 cP and about 4,000 cP, even more particularly between about 1,500 cP and about 2,500 cP, and still more particularly about 2,000 cP;
   (B) providing a substrate comprising at least one skin-contacting surface and configured for insertion into a body cavity, particularly the vagina, mouth, or anus, of one or more of the partners;
   (C) lubricating the at least one skin-contacting surface of the substrate with the antiviral lubricous composition, thereby producing a lubricated substrate;
   (D) contacting the lubricated substrate with the skin or epithelial tissue of one or more of the partners, particularly skin or epithelial tissue located on or within at least one of the vagina, anus, mouth, or penis; and
   (E) transferring the antiviral lubricous composition from the lubricated substrate to at least one of the vagina, anus, mouth, or penis of one or more of the partners, thereby reducing, inhibiting, ameliorating, or preventing the transmission of HPV.
2. The method according to Clause 1, wherein the antiviral lubricous composition further comprises at least one polyol, particularly propylene glycol, comprising less than about 50% by weight of the antiviral lubricous composition, including less than about 25, 15, 10, 9, 8, 7, 6, 5, 4, 3, or 2% by weight, down to less than about 1% by weight of the antiviral lubricous composition.
3. The method according to Clause 2, wherein the antiviral lubricous composition comprises between about 8.0% and about 8.5% by weight of propylene glycol.
4. The method according to Clause 2, wherein the antiviral lubricous composition comprises between about 4.0% and about 4.5% by weight of propylene glycol.
5. The method according to Clause 2, wherein the antiviral lubricous composition comprises between about 2.0% and about 2.5% by weight of propylene glycol.
6. The method of any of Clauses 1-5, wherein the pH of the antiviral lubricous composition is less than about 7.0.
7. The method according to Clause 6, wherein the pH of the antiviral lubricous composition is between about 3.5 and about 5.5, particularly about 4.5.
8. The method according to Clause 6, wherein the pH of the antiviral lubricous composition is between about 5.5 and about 7.0.
9. The method according to any of Clauses 1-8, wherein the antiviral lubricous composition comprises between about 1.4% by weight to about 1.6% by weight of the sea algae extract.
10. The method according to any of Clauses 1-9, wherein the antiviral lubricous composition further comprises a sweetening agent, particularly saccharin, comprising about 0.01% by weight to about 5% by weight of the antiviral lubricous composition, particularly about 0.1% by weight to about 2% by weight of the antiviral lubricous composition.
11. The method according to any of Clauses 1-10, wherein the antiviral lubricous composition further comprises between about 0.01% by weight and about 1.0% by weight of a preservative, particularly a preservative comprising at least one preservative selected from the group consisting of 2-phenoxylethanol, chlorphenesin, and sodium dehydroacetate, including combinations thereof.
12. The method according to any of Clauses 1-11, wherein the substrate is a condom.
13. The method according to Clause 12, wherein the method further comprises the steps of placing a condom over a penis of one partner, lubricating the condom with the antiviral lubricous composition *in situ,* and contacting the skin or epithelial tissue of one or more additional partners.
14. The method according to Clause 12, wherein the method further comprises the steps of sealing the lubricated condom within a packaging, storing the lubricated condom within the packaging, and removing the lubricated condom from the packaging prior to contacting the skin or epithelial tissue of one or more of the partners.
15. The method according to any of Clauses 1-11, wherein the substrate is a sexual accessory device, particularly a sexual accessory device selected from the group consisting of a sex toy, a vibrator, a ring, or beads.
16. The method according to Clause 15, wherein the method further comprises the steps of sealing the lubricated sexual accessory device within a packaging, storing the lubricated sexual accessory device within the packaging, and removing the lubricated sexual accessory device from the packaging prior to contacting the skin or epithelial tissue of one or more of the partners.
17. The method according to any of Clauses 1-16, wherein the lubricated substrate is contacted with the skin or epithelial tissue of one or more of the partners prior to sexual activity, including less than about 8 hours, 4 hours, 2 hours, 1 hour, 30 minutes, 15 minutes, 5 minutes, 1 minute, or about 30 seconds prior to sexual activity, down to less than about 1 second prior to sexual activity.
18. The method according to any of Clauses 1-17, wherein the lubricated substrate is contacted with the skin or epithelial tissue of one or more of the partners after sexual activity, including less than about 8 hours, 4 hours, 2 hours, 1 hour, 30 minutes, 15 minutes, 5 minutes, 1 minute, or about 30 seconds after sexual activity, down to less than about 1 second after sexual activity.
19. A kit for use in reducing, inhibiting, or ameliorating the transmission of HPV between partners engaging in sexual activity, the kit comprising:
   (A) an antiviral lubricous composition comprising:
      (i) a water-soluble sea algae extract comprising lambda-carrageenan, wherein
         (a) lambda-carrageenan comprises at least 50% by weight of the sea algae extract, including at least about 60, 70, 80, or 85 percent by weight, up to at least about 90% by weight of the sea algae extract, particularly between about 80% by weight and about 90% by weight of the sea algae extract, and
         (b) the sea algae extract comprises at least about 0.001% by weight of the antiviral lubricous composition, including at least about 0.01, 0.1, 1, or 3% by weight, up to at least about 5% by weight of the antiviral lubricous composition, particularly between about 1% by weight and about 3% by weight of the antiviral lubricous composition, and
      (ii) a pH-adjusting agent comprising an acid, particularly an organic acid, and more particularly citric acid,
      wherein the antiviral lubricous composition has a viscosity of less than about 10,000 cP, particularly between about 500 cP and about 8,000 cP, more particularly between about 1,000 cP and about 4,000 cP, even more particularly between about 1,500 cP and about 2,500 cP, and still more particularly about 2,000 cP;
   (B) a substrate comprising at least one skin-contacting surface and configured for insertion into a body cavity, particularly the vagina, mouth, or anus, of one or more of the partners; and
   (C) instructions describing the method of any of Clauses 1-18 for contacting the antiviral lubricous composition with the skin of one or more of the partners.
20. The kit according to Clause 19, wherein the antiviral lubricous composition further comprises at least one polyol, particularly propylene glycol, comprising less than about 50% by weight of the antiviral lubricous composition, including less than about 25, 15, 10, 9, 8, 7, 6, 5, 4, 3, or 2% by weight, down to less than about 1% by weight of the antiviral lubricous composition.
21. The kit according to Clause 20, wherein the antiviral lubricous composition comprises between about 8.0% and about 8.5% by weight of propylene glycol.
22. The kit according to Clause 20, wherein the antiviral lubricous composition comprises between about 4.0% and about 4.5% by weight of propylene glycol.
23. The kit according to Clause 20, wherein the antiviral lubricous composition comprises between about 2.0% and about 2.5% by weight of propylene glycol.
24. The kit of any of Clauses 19-23, wherein the pH of the antiviral lubricous composition is less than about 7.0.
25. The kit according to Clause 24, wherein the pH of the antiviral lubricous composition is between about 3.5 and about 5.5, particularly about 4.5.
26. The kit according to Clause 24, wherein the pH of the antiviral lubricous composition is between about 5.5 and about 7.0.
27. The kit according to any of Clauses 19-26, wherein the antiviral lubricous composition comprises between about 1.4% by weight to about 1.6% by weight of the sea algae extract.
28. The kit according to any of Clauses 19-27, wherein the antiviral lubricous composition further comprises a sweetening agent, particularly saccharin, comprising about 0.01% by weight to about 5% by weight of the antiviral lubricous composition, particularly about 0.1% by weight to about 2% by weight of the antiviral lubricous composition.
29. The kit according to any of Clauses 19-28, wherein the antiviral lubricous composition further comprises between about 0.01% by weight and about 1.0% by weight of a preservative, particularly a preservative comprising at least one preservative selected from the group consisting of 2-phenoxylethanol, chlorphenesin, and sodium dehydroacetate, including combinations thereof.
30. The kit according to any of Clauses 19-29, wherein the substrate is a condom.
31. The kit according to Clause 30, wherein the condom is pre-lubricated with the antiviral lubricous composition.
32. The kit according to any of Clauses 19-29, wherein the substrate is a sexual accessory device, particularly a sexual accessory device selected from the group consisting of a sex toy, a vibrator, a ring, or beads.
33. The kit according to Clause 32, wherein the sexual accessory device is pre-lubricated with the antiviral lubricous composition.
34. A method for reducing, inhibiting, or ameliorating the transmission of HPV between partners engaging in sexual activity, the steps comprising:
   (A) providing a pourable, homogeneous antiviral lubricous composition made by a process comprising the steps of:
      (i) providing a water-soluble sea algae extract comprising lambda-carrageenan, wherein the sea algae extract comprises at least 50% by weight lambda-carrageenan; and
      (ii) mixing the water-soluble sea algae extract and a pH-adjusting agent comprising an acid, with an aqueous solution, to form a lubricous composition comprising at least about 0.001% by weight the sea algae extract, wherein the antiviral lubricous composition has a viscosity of less than about 10,000 cP;
   (B) providing a substrate comprising at least one skin-contacting surface and configured for insertion into a body cavity, particularly the vagina, mouth, or anus, of one or more of the partners;
   (C) lubricating the at least one skin-contacting surface of the substrate with the antiviral lubricous composition, thereby producing a lubricated substrate;
   (D) contacting the lubricated substrate with the skin or epithelial tissue of one or more of the partners, particularly skin or epithelial tissue located on or within at least one of the vagina, anus, mouth, or penis; and
   (E) transferring the antiviral lubricous composition from the lubricated substrate to at least one of the vagina, anus, mouth, or penis of one or more of the partners, thereby reducing, inhibiting, ameliorating, or preventing the transmission of HPV.
35. The method according to Clause 34, wherein the antiviral lubricous composition further comprises at least one polyol, particularly propylene glycol, comprising less than about 50% by weight of the antiviral lubricous composition, including less than about 25, 15, 10, 9, 8, 7, 6, 5, 4, 3, or 2% by weight, down to less than about 1% by weight of the antiviral lubricous composition.
36. The method according to Clause 35, wherein the antiviral lubricous composition comprises between about 8.0% and about 8.5% by weight of propylene glycol.
37. The method according to Clause 35, wherein the antiviral lubricous composition comprises between about 4.0% and about 4.5% by weight of propylene glycol.
38. The method according to Clause 35, wherein the antiviral lubricous composition comprises between about 2.0% and about 2.5% by weight of propylene glycol.
39. The method of any of Clauses 34-38, wherein the pH of the antiviral lubricous composition is less than about 7.0.
40. The method according to Clause 39, wherein the pH of the antiviral lubricous composition is between about 3.5 and about 5.5, particularly about 4.5.
41. The method according to Clause 39, wherein the pH of the antiviral lubricous composition is between about 5.5 and about 7.0.
42. The method according to any of Clauses 34-41, wherein the antiviral lubricous composition comprises between about 1.4% by weight to about 1.6% by weight of the sea algae extract.
43. The method according to any of Clauses 34-42, wherein the antiviral lubricous composition further comprises a sweetening agent, particularly saccharin, comprising about 0.01% by weight to about 5% by weight of the antiviral lubricous composition, particularly about 0.1% by weight to about 2% by weight of the antiviral lubricous composition.
44. The method according to any of Clauses 34-43, wherein the antiviral lubricous composition further comprises between about 0.01 % by weight and about 1.0% by weight of a preservative, particularly a preservative comprising at least one preservative selected from the group consisting of 2-phenoxylethanol, chlorphenesin, and sodium dehydroacetate, including combinations thereof.
45. The method according to any of Clauses 34-44, wherein the substrate is a condom.
46. The method according to Clause 45, wherein the method further comprises the steps of placing a condom over a penis of one partner, lubricating the condom with the antiviral lubricous composition *in situ,* and contacting the skin or epithelial tissue of one or more additional partners.
47. The method according to Clause 45, wherein the method further comprises the steps of sealing the lubricated condom within a packaging, storing the lubricated condom within the packaging, and removing the lubricated condom from the packaging prior to contacting the skin or epithelial tissue of one or more of the partners.
48. The method according to any of Clauses 34-44, wherein the substrate is a sexual accessory device, particularly a sexual accessory device selected from the group consisting of a sex toy, a vibrator, a ring, or beads.
49. The method according to Clause 48, wherein the method further comprises the steps of sealing the lubricated sexual accessory device within a packaging, storing the lubricated sexual accessory device within the packaging, and removing the lubricated sexual accessory device from the packaging prior to contacting the skin or epithelial tissue of one or more of the partners.
50. The method according to any of Clauses 34-49, wherein the lubricated substrate is contacted with the skin or epithelial tissue of one or more of the partners prior to sexual activity, including less than about 8 hours, 4 hours, 2 hours, 1 hour, 30 minutes, 15 minutes, 5 minutes, 1 minute, or about 30 seconds prior to sexual activity, down to less than about 1 second prior to sexual activity.
51. The method according to any of Clauses 34-50, wherein the lubricated substrate is contacted with the skin or epithelial tissue of one or more of the partners after sexual activity, including less than about 8 hours, 4 hours, 2 hours, 1 hour, 30 minutes, 15 minutes, 5 minutes, 1 minute, or about 30 seconds after sexual activity, down to less than about 1 second after sexual activity.
52. A kit for use in reducing, inhibiting, or ameliorating the transmission of HPV between partners engaging in sexual activity, the kit comprising:
   (A) a pourable, homogeneous antiviral lubricous composition made by a process comprising the steps of:
      (i) providing a water-soluble sea algae extract comprising lambda-carrageenan, wherein the sea algae extract comprises at least 50% by weight lambda-carrageenan; and
      (ii) mixing the water-soluble sea algae extract and a pH-adjusting agent comprising an acid, with an aqueous solution, to form a lubricous composition comprising at least about 0.001% by weight the sea algae extract, wherein the antiviral lubricous composition has a viscosity of less than about 10,000 cP;
   (B) a substrate comprising at least one skin-contacting surface and configured for insertion into a body cavity, particularly the vagina, mouth, or anus, of one or more of the partners; and
   (C) instructions describing the method of any of Clauses 34-51 for contacting the antiviral lubricous composition with the skin of one or more of the partners.
53. The kit according to Clause 52, wherein the antiviral lubricous composition further comprises at least one polyol, particularly propylene glycol, comprising less than about 50% by weight of the antiviral lubricous composition, including less than about 25, 15, 10, 9, 8, 7, 6, 5, 4, 3, or 2% by weight, down to less than about 1% by weight of the antiviral lubricous composition.
54. The kit according to Clause 53, wherein the antiviral lubricous composition comprises between about 8.0% and about 8.5% by weight of propylene glycol.
55. The kit according to Clause 53, wherein the antiviral lubricous composition comprises between about 4.0% and about 4.5% by weight of propylene glycol.
56. The kit according to Clause 53, wherein the antiviral lubricous composition comprises between about 2.0% and about 2.5% by weight of propylene glycol.
57. The kit of any of Clauses 52-56, wherein the pH of the antiviral lubricous composition is less than about 7.0.
58. The kit according to Clause 57, wherein the pH of the antiviral lubricous composition is between about 3.5 and about 5.5, particularly about 4.5.
59. The kit according to Clause 57, wherein the pH of the antiviral lubricous composition is between about 5.5 and about 7.0.
60. The kit according to any of Clauses 52-59, wherein the antiviral lubricous composition comprises between about 1.4% by weight to about 1.6% by weight of the sea algae extract.
61. The kit according to any of Clauses 52-60, wherein the antiviral lubricous composition further comprises a sweetening agent, particularly saccharin, comprising about 0.01% by weight to about 5% by weight of the antiviral lubricous composition, particularly about 0.1% by weight to about 2% by weight of the antiviral lubricous composition.
62. The kit according to any of Clauses 52-61, wherein the antiviral lubricous composition further comprises between about 0.01% by weight and about 1.0% by weight of a preservative, particularly a preservative comprising at least one preservative selected from the group consisting of 2-phenoxylethanol, chlorphenesin, and sodium dehydroacetate, including combinations thereof.
63. The kit according to any of Clauses 52-62, wherein the substrate is a condom.
64. The kit according to Clause 63, wherein the condom is pre-lubricated with the antiviral lubricous composition.
65. The kit according to any of Clauses 52-62, wherein the substrate is a sexual accessory device, particularly a sexual accessory device selected from the group consisting of a sex toy, a vibrator, a ring, or beads.
66. The kit according to Clause 65, wherein the sexual accessory device is pre-lubricated with the antiviral lubricous composition.
67. A lubricated substrate comprising a skin-contacting surface, wherein the skin-contacting surface of the lubricated substrate is lubricated with an antiviral lubricous composition useful for reducing, inhibiting, or ameliorating the transmission of HPV during sexual activity, the antiviral lubricous composition comprising:
   (A) a water-soluble sea algae extract comprising lambda-carrageenan, wherein
      (i) lambda-carrageenan comprises at least 50% by weight of the sea algae extract, including at least about 60, 70, 80, or 85 percent by weight, up to at least about 90% by weight of the sea algae extract, particularly between about 80% by weight and about 90% by weight of the sea algae extract, and
      (ii) the sea algae extract comprises at least about 0.001% by weight of the antiviral lubricous composition, including at least about 0.01, 0.1, 1, or 3% by weight, up to at least about 5% by weight of the antiviral lubricous composition, particularly between about 1% by weight and about 3% by weight of the antiviral lubricous composition, and
   (B) a pH-adjusting agent comprising an acid, particularly an organic acid, and more particularly citric acid,
   wherein the antiviral lubricous composition has a viscosity of less than about 10,000 cP, particularly between about 500 cP and about 8,000 cP, more particularly between about 1,000 cP and about 4,000 cP, even more particularly between about 1,500 cP and about 2,500 cP, and still more particularly about 2,000 cP.
68. The lubricated substrate according to Clause 67, wherein the antiviral lubricous composition further comprises at least one polyol, particularly propylene glycol, comprising less than about 50% by weight of the antiviral lubricous composition, including less than about 25, 15, 10, 9, 8, 7, 6, 5, 4, 3, or 2% by weight, down to less than about 1% by weight of the antiviral lubricous composition.
69. The lubricated substrate according to Clause 68, wherein the antiviral lubricous composition comprises between about 8.0% and about 8.5% by weight of propylene glycol.
70. The lubricated substrate according to Clause 68, wherein the antiviral lubricous composition comprises between about 4.0% and about 4.5% by weight of propylene glycol.
71. The lubricated substrate according to Clause 68, wherein the antiviral lubricous composition comprises between about 2.0% and about 2.5% by weight of propylene glycol.
72. The lubricated substrate of any of Clauses 67-71, wherein the pH of the antiviral lubricous composition is less than about 7.0.
73. The lubricated substrate according to Clause 72, wherein the pH of the antiviral lubricous composition is between about 3.5 and about 5.5, particularly about 4.5.
74. The lubricated substrate according to Clause 72, wherein the pH of the antiviral lubricous composition is between about 5.5 and about 7.0.
75. The lubricated substrate according to any of Clauses 67-74, wherein the antiviral lubricous composition comprises between about 1.4% by weight to about 1.6% by weight of the sea algae extract.
76. The lubricated substrate according to any of Clauses 67-75, wherein the antiviral lubricous composition further comprises a sweetening agent, particularly saccharin, comprising about 0.01% by weight to about 5% by weight of the antiviral lubricous composition, particularly about 0.1% by weight to about 2% by weight of the antiviral lubricous composition.
77. The lubricated substrate according to any of Clauses 67-76, wherein the antiviral lubricous composition further comprises between about 0.01% by weight and about 1.0% by weight of a preservative, particularly a preservative comprising at least one preservative selected from the group consisting of 2-phenoxylethanol, chlorphenesin, and sodium dehydroacetate, including combinations thereof.
78. The lubricated substrate according to any of Clauses 67-77, wherein the substrate is a condom.
79. The lubricated condom according to Clause 78, further comprising a watertight packaging that seals the lubricated condom from an external environment outside of the packaging.
80. The lubricated substrate according to any of Clauses 67-77, wherein the substrate is a sexual accessory device, particularly a sexual accessory device selected from the group consisting of a sex toy, a vibrator, a ring, or beads.
81. A lubricated substrate comprising a skin-contacting surface, wherein the skin-contacting surface of the lubricated substrate is lubricated with an antiviral lubricous composition useful for reducing, inhibiting, or ameliorating the transmission of HPV during sexual activity, the antiviral lubricous composition made by a process comprising the steps of:
   (A) providing a water-soluble sea algae extract comprising lambda-carrageenan, wherein the sea algae extract comprises at least 50% by weight lambda-carrageenan; and
   (B) mixing the water-soluble sea algae extract and a pH-adjusting agent comprising an acid, with an aqueous solution, to form a lubricous composition comprising at least about 0.001% by weight the sea algae extract, wherein the antiviral lubricous composition has a viscosity of less than about 10,000 cP.
82. The lubricated substrate according to Clause 81, wherein the antiviral lubricous composition further comprises at least one polyol, particularly propylene glycol, comprising less than about 50% by weight of the antiviral lubricous composition, including less than about 25, 15, 10, 9, 8, 7, 6, 5, 4, 3, or 2% by weight, down to less than about 1% by weight of the antiviral lubricous composition.
83. The lubricated substrate according to Clause 82, wherein the antiviral lubricous composition comprises between about 8.0% and about 8.5% by weight of propylene glycol.
84. The lubricated substrate according to Clause 82, wherein the antiviral lubricous composition comprises between about 4.0% and about 4.5% by weight of propylene glycol.
85. The lubricated substrate according to Clause 82, wherein the antiviral lubricous composition comprises between about 2.0% and about 2.5% by weight of propylene glycol.
86. The lubricated substrate of any of Clauses 81-85, wherein the pH of the antiviral lubricous composition is less than about 7.0.
87. The lubricated substrate according to Clause 86, wherein the pH of the antiviral lubricous composition is between about 3.5 and about 5.5, particularly about 4.5.
88. The lubricated substrate according to Clause 86, wherein the pH of the antiviral lubricous composition is between about 5.5 and about 7.0.
89. The lubricated substrate according to any of Clauses 81-88, wherein the antiviral lubricous composition comprises between about 1.4% by weight to about 1.6% by weight of the sea algae extract.
90. The lubricated substrate according to any of Clauses 81-89, wherein the antiviral lubricous composition further comprises a sweetening agent, particularly saccharin, comprising about 0.01% by weight to about 5% by weight of the antiviral lubricous composition, particularly about 0.1% by weight to about 2% by weight of the antiviral lubricous composition.
91. The lubricated substrate according to any of Clauses 81-90, wherein the antiviral lubricous composition further comprises between about 0.01% by weight and about 1.0% by weight of a preservative, particularly a preservative comprising at least one preservative selected from the group consisting of 2-phenoxylethanol, chlorphenesin, and sodium dehydroacetate, including combinations thereof.
92. The lubricated substrate according to any of Clauses 81-91, wherein the substrate is a condom.
93. The lubricated condom according to Clause 92, further comprising a watertight packaging that seals the lubricated condom from an external environment outside of the packaging.
94. The lubricated substrate according to any of Clauses 81-91, wherein the substrate is a sexual accessory device, particularly a sexual accessory device selected from the group consisting of a sex toy, a vibrator, a ring, or beads.

## Claims

1. A pourable, homogeneous, antiviral lubricous composition for use in reducing, inhibiting, ameliorating, or preventing the transmission of human papilloma virus (HPV), wherein the antiviral lubricious composition comprises:
(i) a water-soluble sea algae extract comprising lambda-carrageenan, wherein the sea algae extract comprising at least 50% by weight lambda-carrageenan; and
(ii) a pH-adjusting agent comprising an acid
wherein the antiviral lubricous composition has a viscosity of less than 10,000 cP

2. A pourable, homogeneous, antiviral lubricous composition for use in the treatment of cancer by reducing, inhibiting, ameliorating, or preventing the transmission of human papilloma virus (HPV) , wherein the antiviral lubricious composition comprises:
(i) a water-soluble sea algae extract comprising lambda-carrageenan, wherein the sea algae extract comprising at least 50% by weight lambda-carrageenan; and
(ii) a pH-adjusting agent comprising an acid
wherein the antiviral lubricous composition has a viscosity of less than 10,000 cP

3. The composition of claim 2, wherein the cancer is cervical cancer.

4. The composition of any one of claims 1 to 3, wherein the composition further comprises at least one polyol.

5. The composition of claim 4, wherein the polyol is propylene glycol.

6. The composition of claim 4 or claim 5, wherein the lubricous composition comprises less than 25% by weight of the polyol.

7. The composition of any one of claims 1 to 6, wherein the lubricous composition comprises up to 5% by weight the sea algae extract.

8. The composition of any one of claims 1 to 7, wherein the acid is an organic acid.

9. The composition of claim 8, wherein the organic acid is citric acid.

10. The composition of any one of claims 1 to 9, wherein the lambda-carrageenan comprises between 80% by weight and 90% by weight of the sea algae extract, the sea algae extract comprises between 1% by weight and 3% by weight of the antiviral lubricous composition, and the propylene glycol comprises less than 10% by weight of the antiviral lubricous composition.

11. The composition of any one of claims 1 to 10, wherein the pH of the antiviral lubricous composition is less than 7.0.

12. The composition of any one of claims 1 to 11, wherein the antiviral lubricous composition comprises between 4.0% and 4.5% by weight of propylene glycol.

13. The composition of any one of claims 1 to 12, wherein the antiviral lubricous composition comprises less than 2.2% by weight of the sea algae extract and the viscosity of the antiviral lubricous composition is less than 4,750 cP.

14. The composition of any one of claims 1 to 13, wherein the antiviral lubricous composition further comprises:
a. saccharin, comprising 0.01% by weight to 5% by weight of the antiviral lubricous composition; and/or
b. between 0.01% by weight and 1.0% by weight of a preservative;
optionally, wherein the preservative is selected from the group consisting of: methylparaben; benzoic acid; salicylic acid; sorbic acid; propylparaben; sodium dehydroacetate; and combinations thereof.

15. The composition of any one of claims 1 to 14, wherein the antiviral lubricous composition is a deionized water solution with 4.40 % (w/v) propylene glycol, 0.125% (w/v) sodium saccharin, 1.60 % (w/v) sea algae extract, 0.05 % (w/v) citric acid, 0.32 % (w/v) 2-phenoxyethanol, 0.11 % (w/v) chlorphenesin, and 0.03 % (w/v) sodium dehydroacetate.
